# EUROPÄISCHE PATENTSCHRIFT

(11) **EP 2 313 119 B1**
(45) Veröffentlichungstag und Bekanntmachung des Hinweises auf die Patenterteilung: **26.12.2012**
(21) Anmeldenummer: 09781648.2
(22) Anmeldetag: 10.08.2009
(51) Int. Cl.: A61L 15/60, C08F 2/00, C08K 5/21

(54) **VERFAHREN ZUR HERSTELLUNG VON SUPERABSORBERN MIT NIEDRIGEM RESTMONOMERENGEHALT**
METHOD FOR PRODUCING SUPERABSORBERS WITH A LOW RESIDUAL MONOMER CONTENT
PROCÉDÉ DE PRÉPARATION DE SUPERABSORBANTS AYANT UNE FAIBLE TENEUR EN MONOMÈRES RÉSIDUELS

(30) Priorität: 12.08.2008 EP 08162218
(43) Veröffentlichungstag der Anmeldung: 27.04.2011
(73) Patentinhaber: BASF SE, 67056 Ludwigshafen (DE)
(72) Erfinder: ELLIOTT, Mark, 67063 Ludwigshafen (DE); DANIEL, Thomas, 67165 Waldsee (DE)
(86) Internationale Anmeldenummer: PCT/EP2009/060315
(87) Internationale Veröffentlichungsnummer: WO 2010/018143

(56) Entgegenhaltungen:
- WO-A1-2008/055856

## Beschreibung

Die vorliegende Erfindung betrifft einen Superabsorber mit niedrigem Restmonomerengehalt, ein Verfahren zu seiner Herstellung sowie seine Verwendung und ihn enthaltende Hygieneartikel.

Superabsorber sind bekannt. Für derartige Materialien sind auch Bezeichnungen wie "hochquellfähiges Polymer" "Hydrogel" (oft auch für die trockene Form verwendet), "Hydrogel bildendes Polymer", "Wasser absorbierendes Polymer", "absorbierendes gelbildendes Material", "quellfähiges Harz", "Wasser absorbierendes Harz", Wasser absorbierendes Polymer" oder ähnliche gebräuchlich. Es handelt sich dabei um vernetzte hydrophile Polymere, insbesondere Polymere aus (co)polymerisierten hydrophilen Monomeren, Pfropf(co)polymere von einem oder mehreren hydrophilen Monomeren auf einer geeigneten Pfropfgrundlage, vernetzte Cellulose- oder Stärkeether, vernetzte Carboxymethylcellulose, teilweise vernetztes Polyalkylenoxid oder in wässrigen Flüssigkeiten quellbare Naturprodukte, wie beispielsweise Guarderivate, wobei Superabsorber auf Basis teilneutralisierter Acrylsäure am weitesten verbreitet sind. Die wesentlichen Eigenschaften von Superabsorbern sind ihre Fähigkeiten, ein Vielfaches ihres Eigengewichts an wässrigen Flüssigkeiten zu absorbieren und die Flüssigkeit auch unter gewissem Druck nicht wieder abzugeben. Der Superabsorber, der in Form eines trockenen Pulvers eingesetzt wird, wandelt sich bei Flüssigkeitsaufnahme in ein Gel, bei der üblichen Wasseraufnahme entsprechend in ein Hydrogel um. Die Vernetzung ist für synthetische Superabsorber wesentlich und ein wichtiger Unterschied zu üblichen reinen Verdickern, da sie zur Unlöslichkeit der Polymeren in Wasser führt. Lösliche Substanzen wären als Superabsorber nicht brauchbar. Das mit weitem Abstand wichtigste Einsatzgebiet von Superabsorbern ist das Absorbieren von Körperflüssigkeiten. Superabsorber werden beispielsweise in Windeln für Kleinkinder, Inkontinenzprodukten für Erwachsene oder Damenhygieneprodukten verwendet. Andere Anwendungsgebiete sind beispielsweise die als wasserzurückhaltende Mittel im landwirtschaftlichen Gartenbau, als Wasserspeicher zum Schutz vor Feuer, zur Flüssigkeitsabsorption in Lebensmittelverpackungen oder ganz allgemein zur Absorption von Feuchtigkeit.

Superabsorber können ein mehrfaches ihres Eigengewichts an Wasser absorbieren und unter gewissem Druck zurückhalten. Im Allgemeinen weist ein derartiger Superabsorber eine CRC ("Centrifuge Retention Capacity", Meßmethode siehe unten) von mindestens 5 g/g, vorzugsweise mindestens 10 g/g und in besonders bevorzugter Form mindestens 15 g/g auf. Ein "Superabsorber" kann auch ein Gemisch stofflich verschiedener einzelner Superabsorber sein oder ein Gemisch von Komponenten, die erst im Zusammenwirken superabsorbierende Eigenschaften zeigen, es kommt hier weniger auf die stoffliche Zusammensetzung an als auf die superabsorbierenden Eigenschaften.

Wichtig für einen Superabsorber ist nicht nur seine Absorptionskapazität, sondern auch die Fähigkeit, Flüssigkeit unter Druck zurückzuhalten (Retention) sowie der Flüssigkeitstransport im gequollenen Zustand. Gequollenes Gel kann den Flüssigkeitstransport zu noch nicht gequollenem Superabsorber behindern bis verhindern ("gel blocking"). Gute Transporteigenschaften für Flüssigkeiten besitzen beispielsweise Hydrogele, die im gequollenen Zustand eine hohe Gelfestigkeit aufweisen. Gele mit nur geringer Gelfestigkeit sind unter einem angewendetem Druck (Körperdruck) deformierbar, verstopfen Poren in dem Superabsorber / Cellulosefaser-Saugkörper und verhindern dadurch eine weitere Flüssigkeitsaufnahme. Eine erhöhte Gelfestigkeit wird in aller Regel durch einen höheren Vernetzungsgrad erreicht, wodurch allerdings Absorptionskapazität des Produktes verringert wird. Eine elegante Methode zur Erhöhung der Gelfestigkeit stellt die Erhöhung des Vernetzungsgrads an der Oberfläche der Superabsorberpartikel gegenüber dem Inneren der Partikel dar. Dazu werden meist in einem Oberflächennachvernetzungsschritt getrocknete Superabsorberpartikel mit durchschnittlicher Vernetzungsdichte einer zusätzlichen Vernetzung in einer dünnen Oberflächenschicht ihrer Partikel unterworfen. Durch die Oberflächennachvernetzung steigt die Vernetzungsdichte in der Schale der Superabsorberpartikel, wodurch die Absorption unter Druckbelastung auf ein höheres Niveau gehoben wird. Während die Absorptionskapazität in der Oberflächenschicht der Superabsorberpartikel sinkt, weist ihr Kern durch das Vorhandensein beweglicher Polymerketten eine im Vergleich zur Schale verbesserte Absorptionskapazität auf, so dass durch den Schalenaufbau eine verbesserte Flüssigkeitsweiterleitung gewährleistet wird, ohne dass gel blocking auftritt. Es ist ebenfalls bekannt, insgesamt höher vernetzte Superabsorber zu erzeugen und den Vernetzungsgrad im Inneren der Partikel gegenüber einer äußeren Schale der Partikel nachträglich zu verringern.

Auch Verfahren zur Herstellung von Superabsorbern sind bekannt. Superabsorber auf Basis von Acrylsäure, die auf dem Markt am gängigsten sind, werden durch radikalische Polymerisation von Acrylsäure in Gegenwart eines Vernetzers (dem "Innenvernetzer") hergestellt, wobei die Acrylsäure vor, nach oder teils vor, teils nach der Polymerisation zu einem gewissen Grad neutralisiert wird, üblicherweise durch Zugabe von Alkali, meist einer wässrigen Natriumhydroxidlösung. Das so gewonnene Polymergel wird zerkleinert (je nach verwendetem Polymerisationsreaktor kann dies gleichzeitig mit der Polymerisation erfolgen) und getrocknet. Das so gewonnene trockene Pulver (das "Grundpolymer" oder "Basispolymer") wird üblicherweise an der Oberfläche der Partikel nachvernetzt, indem es mit weiteren Vernetzern wie etwa organischen Vernetzern oder mehrwertigen Kationen, beispielsweise Aluminium (meist als Aluminiumsulfat eingesetzt) oder beidem umgesetzt wird, um eine gegenüber dem Partikelinneren stärker vernetzte Oberflächenschicht zu erzeugen.

Ein bei Superabsorbern oft auftretendes Problem sind Verfärbungen, die beim Lagern unter höherer Temperatur oder höherer Luftfeuchtigkeit auftreten. Derartige Bedingungen treten oft bei Lagerung von Superabsorbern in tropischen oder subtropischen Ländern auf. Unter solchen Bedingungen neigen Superabsorber zum Vergilben, sie können sogar braune oder gar fast schwarze Färbung annehmen. Diese Verfärbung des eigentlich farblosen Superabsorberpulvers ist unansehnlich und unerwünscht, da sie insbesondere bei den erwünschten dünnen Hygieneprodukten sichtbar ist und Verbraucher unansehnliche Hygieneprodukte ablehnen. Die Ursache für die Verfärbung ist nicht vollständig geklärt, allerdings scheinen reaktive Verbindungen wie Restmonomere aus der Polymerisation, Verunreinigungen des Neutralisationsmittels oder der Initiatoren, die Verwendung mancher Initiatoren, Oberflächennachvernetzer oder Stabilisatoren und Verunreinigungen der verwendeten Monomere eine Rolle zu spielen.

Ein weiteres Problem bei der Herstellung von Superabsorbern ist es, unerwünscht hohe Anteile an nicht umgesetzten Monomeren, so genannten Restmonomeren zu vermeiden. Diese können in zu hohen Konzentrationen durchaus toxikologisch bedenklich sein, weiterhin sind sie auch noch reaktiv und können zu unerwünschter Verfärbung führen.

Weiterhin kann ein unerwünscht hoher Gehalt an Eisenionen im Superabsorber zu Problemen führen. Eisenverunreinigungen können zu einem vergleichsweise hohen Gehalt an Restmonomeren und stärkerer Vergilbungsneigung des Superabsorbers führen. Eine häufige Quelle für Eisen in gängigen Superabsorbern ist die Natronlauge, die zur Teilneutralisation von Acrylsäure verwendet wird. Natronlauge ist zwar in verschiedenen Reinheitsgraden erhältlich, die sich auch im Eisengehalt unterscheiden, mit dem Reinheitsgrad steigt jedoch auch der Preis, was den Superabsorber unwirtschaftlicher macht.

Fredric L. Buchholz und Andrew T. Graham (Hrsg.) geben in: "Modern Superabsorbent Polymer Technology", J. Wiley & Sons, New York, U.S.A. / Wiley-VCH, Weinheim, Germany, 1997, ISBN 0-471-19411-5, einen zusammenfassenden Überblick über Superabsorber, ihre Eigenschaften und Verfahren zur Herstellung von Superabsorbern.

WO 2008/055856 A1 lehrt die Vermeidung von Verfärbungen eines Superabsorbers, die durch zu hohen Eisengehalt der Natronlauge hervorgerufen werden, die zur Teilneutralisation der Acrylsäure bei der Herstellung des Superabsorbers verwendet wird, durch Zusatz von Phosphorsäure oder Phosphatsalzen, insbesondere Alkaliphosphaten. JP 05/086 251 A lehrt die Verwendung von Phosphorsäurederivaten oder Salzen davon, insbesondere 1-Hydroxiethyliden-1,1-diphosphonsäure, Ethylendiamin-tetra(methylenphosphonsäure), Diethylentriaminpenta(methylenphosphonsäure) oder deren Alkali- oder Ammoniumsalze als Stabilisatoren von Superabsorbern gegen Verfärbung. WO 00/55245 A1 lehrt die Stabilisierung von Superabsorbern gegen Verfärbung durch Behandlung mit einem anorganischen Reduktionsmittel und wahlweise einem Metallsalz. Das anorganische Reduktionsmittel ist typischerweise ein Hypophosphit, Phosphit, Bisulfit oder Sulfit. Das Metallsalz ist typischerweise ein farbloses (die Eigenschaft "farblos" wird oft auch einfach "weiß" genannt) Phosphat, Acetat oder Läktat, aber kein Halogenid.

Die ältere internationale Patentanmeldung mit Aktenzeichen PCT/EP2008/051009 lehrt den Zusatz eines basischen Salzes eines zweiwertigen Metallkations zu Superabsorbern, unter anderem, um die Stabilität gegen Verfärbung zu erhöhen. Die ältere internationale Patentanmeldung mit dem Aktenzeichen PCT/EP2008/051010 offenbart die Verwendung von Carbonsäuresalzen und/oder basischen Salzen dreiwertiger Metallkationen zum selben Zweck.

DE 38 31 261 A1 lehrt den Zusatz von stickstoffhaltigen, thermisch zersetzbaren Treibmitteln während der Superabsorberherstellung vor der Trocknung, um ein lockeres und gut mahlbares Harz zu gewinnen. Harnstoff wird als mögliches Treibmittel genannt. Nach der Lehre von WO 02/096 953 A1 kann einem Superabsorber auf Polycarboxipolysaccharidbasis ein Treibmittel wie Harnstoff zugesetzt werden, um zusätzliche Porosität zu erzeugen. In WO 2006/119 828 A1 lehrt ein Hybridmaterial aus Polyacrylat-Superabsorber und anorganischen Feststoffteilchen, das als Pflanzensubstrat geeignet ist. Dabei können dem Hybridmaterial, insbesondere dem Superabsorber, bestimmte organische Zusatzstoffe, darunter Harnstoff, entweder als CO₂-Quelle während der Polymerisation, also als Blähmittel, oder auch als düngende Stickstoffquelle zugegeben werden.

WO 98/58 687 A1 lehrt die Verwendung von Harnstoff als einem möglichen Innenvernetzer für Polycarboxipolysaccharid-Superabsorber. WO 2004/018 005 A1 und WO 2004/018 006 A1 nennen Harnstoff als ein mögliches Oberflächennachvernetzungsmittel für Polyacrylat-Superabsorber.

WO 95/17 417 A1 offenbart, dass sich die Flüssigkeitsabsorptionseigenschaften von Polysacchariden durch eine Harnstoffbeschichtung verbessern lassen.
WO 95/19191 A1 nennt Harnstoff in einer Reihe von nichtpolymeren Verbesserungsmitteln, die die Blutabsorption durch Superabsorber verbessern. WO 83/00 289 A1 offenbart Mischungen aus Superabsorber und einem Zusatzstoff, der auch Harnstoff sein kann, als Absorptionsmittel für Blut und seröse Körperflüssigkeiten.

WO 99/26 987 A1 beschreibt die Verringerung von Restmonomeren in Superabsorbern durch Zugabe eines Salzes einer Stickstoffverbindung zur Monomermischung und nachträgliche Erwärmung des Polymerisats bei Temperaturen von 120 bis 240 °C. Als Stickstoffverbindung werden Ammoniak, Hydroxylamin, aliphatische, cycloaliphatische und aromatische Mono- und Polyamine, heterocyclische Amine und Alkanolamine genannt. Das Anion des Salzes ist ein Anion einer anorganischen oder organischen Säure.

Es besteht weiterhin die Aufgabe, ein neues oder verbessertes Verfahren zur Herstellung von Superabsorbern mit niedrigem Restmonomerengehalt und keiner oder höchstens einer tolerablen Färbung. Die Gebrauchseigenschaften des Superabsorbers, insbesondere seine Aufnahmefähigkeit für Flüssigkeit, auch unter Druck, die freie Quellbarkeit sowie seine Fähigkeit zur Weiterleitung von Flüssigkeit, aber auch seine Rieselfähigkeit sollen nicht oder zumindest nicht wesentlich beeinträchtigt werden.

Gelöst wurde die Aufgabe durch ein Polymerisationsverfahren zur Herstellung von Superabsorbern, wobei man der Monomermischung vor oder während der Polymerisation oder dem Polymeren nach der Polymerisation aber vor einer der Polymerisation folgenden Wärmebehandlung ein Salz des Harnstoffs mit einer anorganischen Säure zugibt. Weiterhin wurden der mit diesem Verfahren erhältliche Superabsorber gefunden, Verwendungen dieses Superabsorbers sowie Hygieneartikel, die diesen Superabsorber enthalten.

Das erfindungsgemäße Verfahren führt zu Superabsorbern mit niedrigem Restmonomerengehalt und wenig Verfärbungsneigung, ohne dass ihre Gebrauchseigenschaften beeinträchtigt wären.

Das erfindungsgemäße Polymerisationsverfahren unterscheidet sich von bekannten Polymerisationsverfahren zur Herstellung von Superabsorbern nur dadurch, dass der Monomermischung vor, während oder nach der Polymerisation, aber vor einer der Polymerisation folgenden Wärmebehandlung ein Salz des Harnstoffs mit einer anorganischen Säure zugesetzt wird. Mit anderen Worten, jedes bekannte Polymerisationsverfahren zur Herstellung von Superabsorbern kann durch Zugabe eines Salzes des Harnstoffs mit einer anorganischen Säure in erfindungsgemäßer Weise ausgeübt werden.

Verwendbar ist jedes Salz des Harnstoffs mit einer anorganischen Säure, auch Gemische solcher Salze. Mit anderen Worten: Unter "einem Salz des Harnstoffs mit einer anorganischen Säure" ist auch ein Gemisch solcher Salze zu verstehen. Vorzugsweise wird eine anorganische Säure verwendet, die keine reduzierenden Eigenschaften aufweist, also unter üblichen Bedingungen keine anderen Substanzen reduziert. vorzugsweise wird auch eine nicht oxidierende Säure verwendet, also eine Säure, die unter üblichen Bedingungen keine anderen Substanzen oxidiert. Besonders bevorzugte anorganische Säuren sind insbesondere die Schwefelsäure, die Phosphorsäure, Polyphosporsäuren, die Halogenwasserstoffsäuren, darunter insbesondere die Fluorwasserstoffsäure und die Chlorwasserstoffsäure (Salzsäure), wobei letztere unter den Halogenwasserstoffsäuren bevorzugt ist. Eine ganz besonders bevorzugte Säure ist die Phosphorsäure. Mit anderen Worten: In einer ganz besonders bevorzugten Ausführungsform des erfindungsgemäßen Verfahrens wird vor, während oder nach der Polymerisation, aber vor einem der Polymerisation folgenden Wärmebehandlungsschritt Harnstoffphosphat zugesetzt.

Vorzugsweise erfolgt die Zugabe des Harnstoffsalzes (wobei Gemische von Harnstoffsalzen eingeschlossen sind) mit einer anorganischen Säure vor oder während der Polymerisation, in besonders bevorzugter Weise vor der Polymerisation. Mit anderen Worten, in besonders bevorzugter Weise wird das Salz des Harnstoffs mit einer anorganischen Säure der zu polymerisierenden Monomermischung zugesetzt. Es ist jedoch auch möglich, das Salz erst nach Beginn der Polymerisation zuzugeben oder es dem fertigen Polymer zuzusetzen. Der technische Aufwand, das Salz homogen im Polymer zu verteilen, steigt dabei jedoch, was die Wirtschaftlichkeit beeinträchtigt.

Bei den üblichen Verfahren zur Herstellung von Superabsorbern erfolgt die Polymerisation als Lösungspolymerisation in Wasser oder in einer Suspension von wässriger Monomermischung in einem organischen Suspensionsmittel. Im Anschluss an die Polymerisation ist daher in aller Regel ein Wärmebehandlungsschritt - meist einfach "Trocknung" genannt - erforderlich, um das vorhandene Wasser zu entfernen und trockenen, zur Flüssigkeitsaufnahme befähigten Superabsorber herzustellen. Obwohl bei der Polymerisation Wärme frei wird, wird die Polymerisation selbst nicht als Wärmebehandlungsschritt verstanden. Selbst in Fällen, in denen kein Wasser zu entfernen sein sollte, würde der Superabsorber typischerweise zur Oberflächennachvernetzung einen Wärmebehandlungsschritt durchlaufen. In allen Fällen erfolgt im erfindungsgemäßen Verfahren die Zugabe des Salzes des Harnstoffs mit einer anorganischen Säure vor einem Wärmebehandlungsschritt, vorzugsweise vor der Trocknung. Sollte in einem Spezialfall beim anzuwendenden Verfahren keinerlei Wärmebehandlungsschritt vorgesehen sein, erfolgt die Zugabe des Salzes des Harnstoffs mit einer anorganischen Säure dennoch vor, während oder nach der Polymerisation, vorzugsweise vor oder während der Polymerisation und in besonders bevorzugter Weise vor der Polymerisation.

Im allgemeinen wird das Salz des Harnstoffs mit einer anorganischen Säure in einer Menge von mindestens 0,01 Gew.-%, vorzugsweise von mindestens 0,1 Gew.-%, in besonders bevorzugter Form von mindestens 0,2 Gew.-% und in ganz besonders bevorzugter Form von mindestens 0,3 Gew.-% sowie im Allgemeinen von höchstens 5 Gew.-%, vorzugsweise höchstens 2,5 Gew.-%, in besonders bevorzugter Form höchstens 1,5 Gew.-% und in ganz besonders bevorzugter Form von höchstens 1,0 Gew.-% zugegeben, jeweils bezogen auf den fertigen Superabsorber.

Ein bevorzugtes erfindungsgemäßes Polymerisationsverfahren zur Herstellung von Acrylat-Superabsorbern ist die wässrige Lösungspolymerisation einer Monomermischung, enthaltend
a) mindestens ein ethylenisch ungesättigtes, säuregruppentragendes Monomer, das wahlweise zumindest teilweise als Salz vorliegt,
b) mindestens einen Vernetzer,
c) mindestens einen Initiator,
d) wahlweise ein oder mehrere mit den unter a) genannten Monomeren copolymerisierbare ethylenisch ungesättigte Monomere,
e) wahlweise ein oder mehrere wasserlösliche Polymere und
f) ein Salz des Harnstoffs mit einer anorganischen Säure.

Die Monomeren a) sind vorzugsweise wasserlöslich, d.h. die Löslichkeit in Wasser bei 23°C beträgt typischerweise mindestens 1 g/100 g Wasser, vorzugsweise mindestens 5 g/100 g Wasser, besonders bevorzugt mindestens 25 g/100 g Wasser, ganz besonders bevorzugt mindestens 35 g/100 g Wasser.

Geeignete Monomere a) sind beispielsweise ethylenisch ungesättigte Carbonsäuren oder ihre Salze, wie Acrylsäure, Methacrylsäure, Maleinsäure, Maleinsäureanhydrid, und Itaconsäure oder ihre Salze. Besonders bevorzugte Monomere sind Acrylsäure und Methacrylsäure. Ganz besonders bevorzugt ist Acrylsäure.

Weitere geeignete Monomere a) sind beispielsweise ethylenisch ungesättigte Sulfonsäuren, wie Styrolsulfonsäure und 2-Acrylamido-2-methylpropansulfonsäure (AMPS).

Verunreinigungen können einen erheblichen Einfluss auf die Polymerisation haben. Daher sollten die eingesetzten Rohstoffe eine möglichst hohe Reinheit aufweisen. Es ist daher oft vorteilhaft die Monomeren a) speziell zu reinigen. Geeignete Reinigungsverfahren werden beispielsweise in der WO 2002/055469 A1, der WO 2003/078378 A1 und der WO 2004/035514 A1 beschrieben. Ein geeignetes Monomer a) ist beispielsweise eine gemäß WO 2004/035514 A1 gereinigte Acrylsäure mit 99,8460 Gew.-% Acrylsäure, 0,0950 Gew.-% Essigsäure, 0,0332 Gew.-% Wasser, 0,0203 Gew.-% Propionsäure, 0,0001 Gew.-% Furfurale, 0,0001 Gew.-% Maleinsäureanhydrid, 0,0003 Gew.-% Diacrylsäure und 0,0050 Gew.-% Hydrochinonmonomethylether.

Der Anteil an Acrylsäure und/oder deren Salzen an der Gesamtmenge der Monomeren a) beträgt vorzugsweise mindestens 50 mol-%, besonders bevorzugt mindestens 90 mol-%, ganz besonders bevorzugt mindestens 95 mol-%.

Die Monomerlösung enthält vorzugsweise höchstens 250 Gew.-ppm, bevorzugt höchstens 130 Gew.-ppm, besonders bevorzugt höchstens 70 Gew.-ppm sowie bevorzugt mindestens 10 Gew.-ppm, besonders bevorzugt mindestens 30 Gew.-ppm, insbesondere um 50 Gew.-ppm, Hydrochinonhalbether, jeweils bezogen auf das unneutralisierte Monomer a), wobei neutralisiertes Monomer a), d.h. ein Salz des Monomers a) rechnerisch als unneutralisiertes Monomer berücksichtigt wird. Beispielsweise kann zur Herstellung der Monomerlösung ein ethylenisch ungesättigtes, säuregruppentragendes Monomer mit einem entsprechenden Gehalt an Hydrochinonhalbether verwendet werden.

Bevorzugte Hydrochinonhalbether sind Hydrochinonmonomethylether (MEHQ) und/oder alpha-Tocopherol (Vitamin E).

Geeignete Vernetzer b) sind Verbindungen mit mindestens zwei zur Vernetzung geeigneten Gruppen. Derartige Gruppen sind beispielsweise ethylenisch ungesättigte Gruppen, die in die Polymerkette radikalisch einpolymerisiert werden können, und funktionelle Gruppen, die mit den Säuregruppen des Monomeren a) kovalente Bindungen ausbilden können. Weiterhin sind auch polyvalente Metallsalze, die mit mindestens zwei Säuregruppen des Monomeren a) koordinative Bindungen ausbilden können, als Vernetzer b) geeignet.

Vernetzer b) sind vorzugsweise Verbindungen mit mindestens zwei polymerisierbaren Gruppen, die in das Polymernetzwerk radikalisch einpolymerisiert werden können. Geeignete Vernetzer b) sind beispielsweise Ethylenglykoldimethacrylat, Diethylenglykoldiacrylat, Polyethylenglykoldiacrylat, Allylmethacrylat, Trimethylolpropantriacrylat, Triallylamin, Tetraallylammoniumchlorid, Tetraallyloxyethan, wie in EP 530 438 A1 beschrieben, Di- und Triacrylate, wie in EP 547 847 A1, EP 559 476 A1, EP 632 068 A1, WO 93/21237 A1, WO 2003/104299 A1, WO 2003/104300 A1, WO 2003/104301 A1 und DE 103 31 450 A1 beschrieben, gemischte Acrylate, die neben Acrylatgruppen weitere ethylenisch ungesättigte Gruppen enthalten, wie in DE 103 31 456 A1 und DE 103 55 401 A1 beschrieben, oder Vernetzermischungen, wie beispielsweise in DE 195 43 368 A1, DE 196 46 484 A1, WO 90/15830 A1 und WO 2002/32962 A2 beschrieben.

Bevorzugte Vernetzer b) sind Pentaerythrittriallylether, Tetraallyloxiethan, Methylenbismethacrylamid, 10 bis 20-fach ethoxyliertes Trimethylolpropantriacrylat, 10 bis 20-fach ethoxyliertes Trimethylolethantriacrylat, besonders bevorzugt 15-fach ethoxiliertes Trimethylolpropantriacrylat, Polyethylenglykoldiacrylate mit 4 bis 30 Ethylenoxideinheiten in der Polyethylenglykolkette , Trimethylolpropantriacrylat, Di- und Triacrylate des 3 bis 30-fach ethoxylierten Glycerins, besonders bevorzugt Di- und Triacrylate des 10-20-fach ethoxylierten Glycerins, und Triallylamin. Die nicht vollständig mit Acrylsäure veresterten Polyole können hier auch als Michaeladdukte mit sich selbst vorliegen, wodurch auch tetra-, penta- oder noch höhere Acrylate vorliegen können.

Ganz besonders bevorzugte Vernetzer b) sind die mit Acrylsäure oder Methacrylsäure zu Di- oder Triacrylaten veresterten mehrfach ethoxylierten und/oder propoxylierten Glyzerine, wie sie beispielsweise in WO 2003/104301 A1 beschrieben sind. Besonders vorteilhaft sind Di- und/oder Triacrylate des 3- bis 10-fach ethoxylierten Glyzerins. Ganz besonders bevorzugt sind Di- oder Triacrylate des 1- bis 5-fach ethoxylierten und/oder propoxylierten Glyzerins. Am meisten bevorzugt sind die Triacrylate des 3-bis 5-fach ethoxylierten und/oder propoxylierten Glyzerins, insbesondere das Triacrylat des 3-fach ethoxylierten Glyzerins.

Die Menge an Vernetzer b) beträgt vorzugsweise 0,05 bis 1,5 Gew.-%, besonders bevorzugt 0,1 bis 1 Gew.-%, ganz besonders bevorzugt 0,3 bis 0,6 Gew.-%, jeweils bezogen auf Monomer a). Mit steigendem Vernetzergehalt sinkt die Zentrifugenretentionskapazität (CRC) und steigt die Absorption unter einem Druck von 0.3 psi (AUL0.3psi).

Als Initiatoren c) können sämtliche unter den Polymerisationsbedingungen Radikale erzeugende Verbindungen eingesetzt werden, beispielsweise thermische Initiatoren, Redox-Initiatoren, Photoinitiatoren. Geeignete Redox-Initiatoren sind Natriumperoxodisulfat/Ascorbinsäure, Wasserstoffperoxid/Ascorbinsäure, Natriumperoxodisulfat/Natriumbisulfit und Wasserstoffperoxid/Natriumbisulfit. Vorzugsweise werden Mischungen aus thermischen Initiatoren und Redox-Initiatoren eingesetzt, wie Natriumperoxodisulfat/Wasserstoffperoxid/Ascorbinsäure. Als reduzierende Komponente wird aber vorzugsweise ein Gemisch aus dem Natriumsalz der 2-Hydroxy-2-sulfinato-essigsäure, dem Dinatriumsalz der 2-Hydroxy-2-sulfonatoessigsäure und Natriumbisulfit eingesetzt (als Brüggolit^{®} FF6M oder Brüggolit^{®} FF7, alternativ BRUGGOLITE^{®} FF6M oder BRUGGOLITE^{®} FF7 erhältlich von L. Brüggemann KG, Salzstraße 131, 74076 Heilbronn, Deutschland, www.brueggemann.com).

Mit den ethylenisch ungesättigten, säuregruppentragenden Monomeren a) copolymerisierbare ethylenisch ungesättigte Monomere d) sind beispielsweise Acrylamid, Methacrylamid, Hydroxyethylacrylat, Hydroxyethylmethacrylat, Dimethylaminoethylmethacrylat, Dimethylaminoethylacrylat, Dimethylaminopropylacrylat, Diethylaminopropylacrylat, Dimethylaminoethylmethacrylat, Diethylaminoethylmethacrylat, Maleinsäure und Maleinsäureanhydrid.

Als wasserlösliche Polymere e) können Polyvinylalkohol, Polyvinylpyrrolidon, Stärke, Stärkederivate, modifizierte Cellulose, wie Methylcellulose oder Hydroxyethylcellulose, Gelatine, Polyglykole oder Polyacrylsäuren, vorzugsweise Stärke, Stärkederivate und modifizierte Cellulose, eingesetzt werden.

Salze des Harnstoffs mit einer anorganischen Säure f) sind oben beschrieben. Insbesondere sind hier Salze des Harnstoffs mit Schwefelsäure, Phosphorsäure, Halogenwasserstoffsäuren, darunter insbesondere Fluorwasserstoffsäure und Chlorwasserstoffsäure (Salzsäure), wobei letztere unter den Halogenwasserstoffsäuren bevorzugt ist, geeignet. In ganz besonders bevorzugter Form wird Harnstoffphosphat zugesetzt.

Üblicherweise wird eine wässrige Monomerlösung verwendet. Der Wassergehalt der Monomerlösung beträgt vorzugsweise von 40 bis 75 Gew.-%, besonders bevorzugt von 45 bis 70 Gew.-%, ganz besonders bevorzugt von 50 bis 65 Gew.-%. Es ist auch möglich Monomersuspensionen, d.h. übersättigte Monomerlösungen einzusetzen. Mit steigendem Wassergehalt steigt der Energieaufwand bei der anschließenden Trocknung und mit sinkendem Wassergehalt kann die Polymerisationswärme nur noch ungenügend abgeführt werden.

Die bevorzugten Polymerisationsinhibitoren benötigen für eine optimale Wirkung gelösten Sauerstoff. Daher kann die Monomerlösung vor der Polymerisation durch Inertisierung, d.h. Durchströmen mit einem inerten Gas, vorzugsweise Stickstoff oder Kohlendioxid, von gelöstem Sauerstoff befreit werden. Vorzugsweise wird der Sauerstoffgehalt der Monomerlösung vor der Polymerisation auf weniger als 1 Gew.-ppm, besonders bevorzugt auf weniger als 0,5 Gew.-ppm, ganz besonders bevorzugt auf weniger als 0,1 Gew.-ppm, gesenkt.

Die Monomermischung kann weitere Komponenten enthalten. Beispiele in derartigen Monomermischungen verwendeter weiterer Komponenten sind etwa Chelatbildner, um Metallionen in Lösung zu halten.

Geeignete Polymerisationsreaktoren sind beispielsweise Knetreaktoren oder Bandreaktoren. Im Kneter wird das bei der Polymerisation einer wässrigen Monomerlösung oder -suspension entstehende Polymergel durch beispielsweise gegenläufige Rührwellen kontinuierlich zerkleinert, wie in WO 2001/38402 A1 beschrieben. Die Polymerisation auf dem Band wird beispielsweise in DE 38 25 366 A1 und US 6,241,928 beschrieben. Bei der Polymerisation in einem Bandreaktor entsteht ein Polymergel, das in einem weiteren Verfahrensschritt zerkleinert werden muss, beispielsweise in einem Fleischwolf, Extruder oder Kneter. Es können aber auch sphärische Superabsorberpartikel durch Suspensions-, Sprüh- oder Tropfenpolymerisationsverfahren hergestellt werden. Der Einsatz des erfindungsgemäß besonders bevorzugten Harnstoffphosphats ist insbesondere bei Polymerisationsverfahren wie beispielsweise einem Knetreaktor oder einer Tropfenpolymerisation mit relativ kurzer Polymerisationszeit besonders vorteilhaft.

Die Säuregruppen der erhaltenen Polymergele sind üblicherweise teilweise neutralisiert. Die Neutralisation wird vorzugsweise auf der Stufe der Monomeren durchgeführt, mit anderen Worten, es werden Salze der säuregruppentragenden Monomeren oder genaugenommen eine Mischung von säuregruppentragenden Monomeren und Salzen der säuregruppentragenden Monomeren ("teilneutralisierte Säure") als Komponente a) in die Polymerisation eingesetzt. Dies geschieht üblicherweise durch Einmischung des Neutralisationsmittels als wässrige Lösung oder bevorzugt auch als Feststoff in die zur Polymerisation vorgesehene Monomermischung oder bevorzugt in das säuregruppentragende Monomer oder eine Lösung davon. Der Neutralisationsgrad beträgt vorzugsweise von 25 bis 95 mol-%, besonders bevorzugt von 50 bis 80 mol-%, ganz besonders bevorzugt von 65 bis 72 mol-%, wobei die üblichen Neutralisationsmittel verwendet werden können, vorzugsweise Alkalimetallhydroxide, Alkalimetalloxide, Alkalimetallkarbonate oder Alkalimetallhydrogenkarbonate sowie deren Mischungen. Statt Alkalimetallsalzen können auch Ammoniumsalze verwendet werden. Natrium und Kalium sind als Alkalimetallkationen besonders bevorzugt, ganz besonders bevorzugt sind jedoch Natriumhydroxid, Natriumkarbonat oder Natriumhydrogenkarbonat sowie deren Mischungen.

Es ist aber auch möglich die Neutralisation nach der Polymerisation auf der Stufe des bei der Polymerisation entstehenden Polymergels durchzuführen. Weiterhin ist es möglich bis zu 40 mol-%, vorzugsweise 10 bis 30 mol-%, besonders bevorzugt 15 bis 25 mol-%, der Säuregruppen vor der Polymerisation zu neutralisieren indem ein Teil des Neutralisationsmittels bereits der Monomerlösung zugesetzt und der gewünschte Endneutralisationsgrad erst nach der Polymerisation auf der Stufe des Polymergels eingestellt wird. Wird das Polymergel zumindest teilweise nach der Polymerisation neutralisiert, so wird das Polymergel vorzugsweise mechanisch zerkleinert, beispielsweise mittels eines Extruders, wobei das Neutralisationsmittel aufgesprüht, übergestreut oder aufgegossen und dann sorgfältig untergemischt werden kann. Dazu kann die erhaltene Gelmasse noch mehrmals zur Homogenisierung extrudiert werden.

Es ist jedoch bevorzugt, die Neutralisation auf der Stufe des Monomeren durchzuführen. Mit anderen Worten: in einer ganz besonders bevorzugten Ausführungsform wird als Monomer a) ein Gemisch aus 25 bis 95 mol-%, besonders bevorzugt von 50 bis 80 mol-%, ganz besonders bevorzugt von 65 bis 72 mol-% Salz des säuregruppentragenden Monomeren und dem Rest zu 100 mol-% säuregruppentragendes Monomer eingesetzt. Dieses Gemisch ist beispielsweise ein Gemisch aus Natriumacrylat und Acrylsäure oder ein Gemisch aus Kaliumacrylat und Acrylsäure.

In einer bevorzugten Ausführungsform wird zur Neutralisation ein Neutralisationsmittel verwendet, dessen Gehalt an Eisen im Allgemeinen unter 10 Gew.-ppm, vorzugsweise unter 2 Gew.-ppm und in besonders bevorzugter Weise unter 1 Gew.-ppm liegt. Ebenso ist ein niedriger Gehalt an Chlorid sowie Anionen von Sauerstoffsäuren des Chlors erwünscht. Ein geeignetes Neutralisationsmittel ist beispielsweise die üblicherweise als "membrane grade" gehandelte 50 Gew.-%ige Natronlauge oder Kalilauge, noch reiner und bevorzugt, allerdings auch kostspieliger ist die üblicherweise als "amalgame grade" oder "mercury process" gehandelte 50 Gew.-%ige Natronlauge oder Kalilauge.

Das aus der wässrigen Lösungspolymerisation und gegebenenfalls nachträglicher Neutralisation erhaltene Polymergel wird dann vorzugsweise mit einem Bandtrockner getrocknet, bis der Restfeuchtegehalt vorzugsweise 0,5 bis 15 Gew.-%, besonders bevorzugt 1 bis 10 Gew.-%, ganz besonders bevorzugt 2 bis 8 Gew.-%, beträgt (Messmethode für den Restfeuchte- oder Wassergehalt siehe unten). Bei einer zu hohen Restfeuchte weist das getrocknete Polymergel eine zu niedrige Glasübergangstemperatur Tg auf und ist nur schwierig weiter zu verarbeiten. Bei einer zu niedrigen Restfeuchte ist das getrocknete Polymergel zu spröde und in den anschließenden Zerkleinerungsschritten fallen unerwünscht große Mengen an Polymerpartikeln mit zu niedriger Partikelgröße ("fines") an. Der Feststoffgehalt des Gels beträgt vor der Trocknung im Allgemeinen von 25 bis 90 Gew.-%, vorzugsweise von 30 bis 80 Gew.-%, besonders bevorzugt von 35 bis 70 Gew.-%, ganz besonders bevorzugt von 40 bis 60 Gew.-%. Wahlweise kann zur Trocknung aber auch ein Wirbelbetttrockner oder ein beheizbarer Mischer mit mechanischem Mischorgan wie beispielsweise ein Schaufeltrockner oder ein ähnlicher Trockner mit anders gestalteten Mischwerkzeugen verwendet werden. Wahlweise kann der Trockner unter Stickstoff oder einem anderen nicht-oxidierenden Inertgas oder zumindest unter verringertem Partialdruck des Sauerstoffs betrieben werden, um oxidative Vergilbungsvorgänge zu verhindern. Im Regelfall führt aber auch eine ausreichende Belüftung und Abführung des Wasserdampfes zu einem akzeptablen Produkt. Vorteilhaft hinsichtlich Farbe und Produktqualität ist in der Regel eine möglichst kurze Trocknungszeit. Bei den gängigen Bandtrocknern wird bei üblicher Betriebsweise dazu eine Temperatur des zur Trocknung verwendeten Gases von mindestens 50 °C, vorzugsweise mindestens 80 °C und in besonders bevorzugter Form von mindestens 100 °C sowie im Allgemeinen von höchstens 250 °C, vorzugsweise höchstens 200 °C und in besonders bevorzugter Form von höchstens 180 °C eingestellt. Gängige Bandtrockner haben oft mehrere Kammern, die Temperatur in diesen Kammern kann unterschiedlich sein. Bei jedem Trocknertyp sind die Betriebsbedingungen insgesamt so in bekannter Weise zu wählen, dass das gewünschte Trocknungsergebnis erreicht wird.

Während der Trockung verringert sich auch der Restmonomerengehalt in den Polymerpartikeln und letzte Reste des Initiators werden zerstört.

Das getrocknete Polymergel wird hiernach gemahlen und klassiert, wobei zur Mahlung üblicherweise ein- oder mehrstufige Walzenstühle, bevorzugt zwei- oder dreistufige Walzenstühle, Stiftmühlen, Hammermühlen oder Schwingmühlen eingesetzt werden können. Übergroße, oft im Inneren noch nicht getrocknete Gelklumpen sind gummielastisch, führen zu Problemen bei der Mahlung und werden vorzugsweise vor der Mahlung abgetrennt, was durch Windsichtung oder ein Sieb ("Schutzsieb" für die Mühle) in einfacher Weise erfolgen kann. Die Maschenweite des Siebs ist angesichts der verwendeten Mühle so zu wählen, dass möglichst keine Störungen durch übergroße, gummielastische Partikel auftreten.

Zu große, nicht ausreichend fein gemahlene Superabsorberpartikel sind bei ihrer überwiegenden Verwendung, in Hygieneprodukten wie Windeln, als grobe Partikel fühlbar, sie senken auch die mittlere Anquellgeschwindigkeit des Superabsorbers Beides ist unerwünscht. Vorteilhafterweise werden daher grobkörnige Polymerpartikel aus dem Produkt abgetrennt. Dies erfolgt durch übliche Klassierverfahren, beispielsweise Windsichtung oder durch Siebung durch ein Sieb mit einer Maschenweite von höchstens 1000 µm, vorzugsweise höchstens 900 µm, besonders bevorzugt höchstens 850 µm und ganz besonders bevorzugt höchstens 800 µm. Beispielsweise werden Siebe mit 700 µm, 650 µm oder 600 µm Maschenweite verwendet. Die abgetrennten grobkörnigen Polymerpartikel ("Überkorn") können zur Kostenoptimierung dem Mahl- und Siebkreislauf wieder zugeführt oder separat weiter verarbeitet werden.

Polymerpartikel mit zu niedriger Partikelgröße senken die Permeabilität (SFC). Vorteilhafterweise werden daher bei dieser Klassierung auch feinkörnige Polymerpartikel abgetrennt. Dies kann, falls gesiebt wird, bequem durch ein Sieb mit einer Maschenweite von höchstens 300 µm, vorzugsweise höchstens 200 µm, in besonders bevorzugter Weise höchstens 150 µm und in ganz besonders bevorzugter Weise höchstens 100 µm verwendet. Die abgetrennten feinkörnigen Polymerpartikel ("Unterkorn" oder "fines") können zur Kostenoptimierung beliebig dem Monomerstrom, dem polymerisierenden Gel, oder dem auspolymerisierten Gel vor der Trocknung des Gels wieder zugeführt werden.

Die mittlere Partikelgröße der als Produktfraktion abgetrennten Polymerpartikel beträgt im Allgemeinen mindestens 200 µm, bevorzugt mindestens 250 µm und in bevorzugter Form mindestens 300 µm sowie im Allgemeinen höchstens 600 µm und in bevorzugter Weise höchstens 500 µm. Der Anteil an Partikeln mit einer Partikelgröße von mindestens 150 µm beträgt im Allgemeinen mindestens 90 Gew.-%, bevorzugterweise mindestens 95 Gew.-% und in besonders bevorzugter Weise mindestens 98 Gew.-%. Der Anteil an Partikeln mit einer Partikelgröße von höchstens 850 µm, beträgt im Allgemeinen mindestens 90 Gew.-%, bevorzugterweise mindestens 95 Gew.-% und in besonders bevorzugter Weise mindestens 98 Gew.-%.

Das so hergestellte Polymer hat superabsorbierende Eigenschaften und fällt unter den Begriff "Superabsorber". Seine CRC ist typischerweise vergleichsweise hoch, seine AUL oder SFC dagegen vergleichsweise niedrig. Ein derartiger, nicht oberflächennachvernetzter Superabsorber wird zur Unterscheidung von einem daraus hergestellten oberflächennachvernetzten Superabsorber oft "Grundpolymer" oder "Basispolymer" genannt.

Die Superabsorberpartikel können zur weiteren Verbesserung der Eigenschaften, insbesondere Erhöhung der AUL und SFC-Werte (wobei der CRC-Wert sinkt) an ihrer Oberfläche nachvernetzt werden. Geeignete Nachvernetzer sind Verbindungen, die Gruppen enthalten, die mit mindestens zwei funktionellen Gruppen der Superabsorberpartikel Bindungen bilden können. Bei den auf dem Markt vorherrschenden Superabsorbern auf Acrylsäure/Natriumacrylat-Basis sind geeignete Oberflächennachvernetzer Verbindungen, die Gruppen enthalten, die mit mindestens zwei Carboxylatgruppen Bindungen bilden können. Bevorzugte Nachvernetzer sind Amidacetale oder Carbamate der allgemeinen Formel (I) 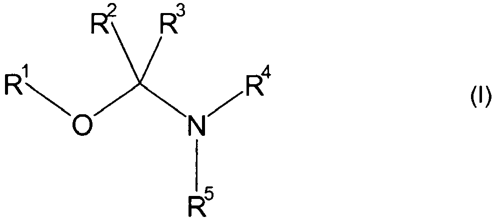 worin
- R¹: C₁-C₁₂-Alkyl, C₂-C₁₂-Hydroxyalkyl, C₂-C₁₂-Alkenyl oder C₆-C₁₂-Aryl,
- R²: X oder OR⁶,
- R³: Wasserstoff, C₁-C₁₂-Alkyl, C₂-C₁₂-Hydroxyalkyl, C₂-C₁₂-Alkenyl oder C₆-C₁₂-Aryl, oder X,
- R⁴: C₁-C₁₂-Alkyl, C₂-C₁₂-Hydroxyalkyl, C₂-C₁₂-Alkenyl oder C₆-C₁₂-Aryl,
- R⁵: Wasserstoff, C₁-C₁₂-Alkyl, C₂-C₁₂-Hydroxyalkyl, C₂-C₁₂-Alkenyl, C₁-C₁₂-Acyl oder C₆-C₁₂-Aryl,
- R⁶: C₁-C₁₂-Alkyl, C₂-C₁₂-Hydroxyalkyl, C₂-C₁₂-Alkenyl oder C₆-C₁₂-Aryl und
- X: ein für die Reste R² und R³ gemeinsamer Carbonylsauerstoff
bedeuten, wobei R¹ und R⁴ und/oder R⁵ und R⁶ ein verbrücktes C₂-C₆-Alkandiyl sein können, und wobei die obengenannte Reste R¹ bis R⁶ noch insgesamt über ein bis zwei freie Valenzen verfügen können und mit diesen freien Valenzen mit mindestens einem geeigneten Grundkörper verbunden sein können,
oder mehrwertige Alkohole, wobei der mehrwertige Alkohol vorzugsweise ein Molekulargewicht von weniger als 100 g/mol, bevorzugt von weniger als 90 g/mol, besonders bevorzugt von weniger als 80 g/mol, ganz besonders bevorzugt von weniger als 70 g/mol, pro Hydroxygruppe sowie keine vicinalen, geminalen, sekundären oder tertiären Hydroxygruppen aufweist, und mehrwertige Alkohole entweder Diole der allgemeinen Formel (IIa)

HO-R⁷-OH (IIa)

worin R⁷ entweder einen unverzweigten Dialkylrest der Formel -(CH₂)ₙ-, wobei n eine ganze Zahl von 3 bis 20, bevorzugt 3 bis 12 ist, bedeutet und beide Hydroxygruppen endständig sind, oder R⁷ einen unverzweigten, verzweigten oder cyclischen Dialkylrest bedeutet, oder Polyole der allgemeinen Formel (IIb) 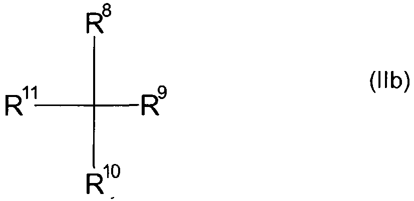 worin die Reste R⁸, R⁹, R¹⁰, R¹¹ unabhängig voneinander Wasserstoff, Hydroxyl, Hydroxymethyl, Hydroxyethyloxymethyl, 1-Hydroxyprop-2-yloxymethyl, 2-Hydroxypropyloxymethyl, Methyl, Ethyl, n-Propyl, Isopropyl, n-Butyl, n-Pentyl, n-Hexyl, 1,2-Dihydroxyethyl, 2-Hydroxyethyl, 3-Hydroxypropyl oder 4-Hydroxybutyl bedeuten und insgesamt 2, 3, oder 4, bevorzugt 2 oder 3, Hydroxygruppen vorhanden sind, und nicht mehr als einer der Reste R⁸, R⁹, R¹⁰, oder R¹¹ gleich Hydroxyl bedeutet, sind,
oder cyclische Carbonate der allgemeinen Formel (III) 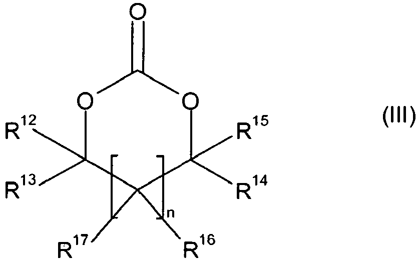 worin R¹², R¹³, R¹⁴, R¹⁵, R¹⁶ und R¹⁷ unabhängig voneinander Wasserstoff, Methyl, Ethyl, n-Propyl, Isopropyl, n-Butyl, sek.-Butyl oder Isobutyl, und n entweder 0 oder 1 ist,
oder Bisoxazoline der allgemeinen Formel (IV) 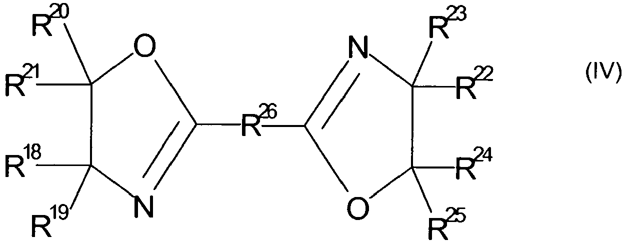 worin R¹⁸, R¹⁹, R²⁰, R²¹, R²², R²³, R²⁴ und R²⁵ unabhängig voneinander Wasserstoff, Methyl, Ethyl, n-Propyl, Isopropyl, n-Butyl, sek.-Butyl oder Isobutyl, und R²⁶ eine Einfachbindung, einen linearen, verzweigten oder cyclischen C₂-C₁₂-Dialkylrest, oder einen Polyalkoxydiylrest darstellt, welcher aus ein bis zehn Ethylenoxid- und/oder Propylenoxideinheiten aufgebaut ist, wie sie beispielsweise Polyglykoldicarbonsäuren aufweisen.

Bevorzugte Nachvernetzer der allgemeinen Formel (II) sind 2-Oxazolidone, wie 2-Oxazolidon und N-(2-Hydroxyethyl)-2-oxazolidon, N-Methyl-2-Oxazolidon, N-Acyl-2-oxazolidone, wie N-Acetyl-2-oxazolidon, 2-Oxotetrahydro-1,3-oxazin, bicyclische Amidacetale, wie 5-Methyl-1-aza-4,6-dioxa-bicyclo[3.3.0]octan, 1-Aza-4,6-dioxa-bicyclo[3.3.0]octan und 5-Isopropyl-1-aza-4,6-dioxa-bicyclo [3.3.0] octan, Bis-2-oxazolidone und Poly-2-oxazolidone.

Besonders bevorzugte Nachvernetzer der allgemeinen Formel (I) sind 2-Oxazolidon, N-Methyl-2-oxazolidon, N-(2-Hydroxyethyl)-2-oxazolidon und N-Hydroxypropyl-2-oxazolidon.

Bevorzugte Nachvernetzer der allgemeinen Formel (IIa) sind 1,3-Propandiol, 1,5-Pentandiol, 1,6-Hexandiol und 1,7-Heptandiol. Weitere Beispiele für Nachvernetzer der Formel (IIa) sind 1,3-Butandiol, 1,8-Octandiol, 1,9-Nonandiol und 1,10-Decandiol.

Die Diole sind vorzugsweise wasserlöslich, wobei sich die Diole der allgemeinen Formel (IIa) bei 23°C zu mindestens 30 Gew.-%, bevorzugt zu mindestens 40 Gew.-%, besonders bevorzugt zu mindestens 50 Gew.-%, ganz besonders bevorzugt mindestens zu 60 Gew.-%, in Wasser lösen, wie beispielsweise 1,3-Propandiol und 1,7-Heptandiol. Noch mehr bevorzugt sind solche Nachvernetzer die bei 25°C flüssig sind.

Bevorzugte Nachvernetzer der allgemeinen Formel (IIb) sind Butan-1,2,3-triol, Butan-1,2,4-triol, Glyzerin, Trimethylolpropan, Trimethylolethan, Pentaerythrit, pro Molekül 1-bis 3-fach ethoxyliertes Glyzerin, Trimethylolethan oder Trimethylolpropan und pro Molekül 1- bis 3-fach propoxyliertes Glyzerin, Trimethylolethan oder Trimethylolpropan.

Weiterhin bevorzugt sind 2-fach ethoxyliertes oder propoxyliertes Neopentylglykol. Besonders bevorzugt sind 2-fach und 3-fach ethoxyliertes Glyzerin, Neopentylglykol, 2-Methyl-1,3-propandiol und Trimethylolpropan.

Bevorzugte mehrwertige Alkohole (IIa) und (IIb) weisen bei 23 °C eine Viskosität von weniger als 3000 mPas, vorzugsweise weniger als 1500 mPas, bevorzugt weniger als 1000 mPas, besonders bevorzugt weniger als 500 mPas, ganz besonders bevorzugt weniger als 300 mPas, auf.

Besonders bevorzugte Nachvernetzer der allgemeinen Formel (III) sind Ethylencarbonat und Propylencarbonat.

Ein besonders bevorzugter Nachvernetzer der allgemeinen Formel (IV) ist 2,2'-Bis(2-oxazolin).

Die bevorzugten Nachvernetzer minimieren Neben- und Folgereaktionen, die zu flüchtigen und damit übelriechenden Verbindungen führen. Die mit den bevorzugten Nachvernetzern hergestellten Superabsorber sind daher auch im angefeuchteten Zustand geruchsneutral.

Es kann ein einzelner Nachvernetzer aus der obigen Auswahl verwendet werden oder beliebige Gemische verschiedener Nachvernetzer.

Der Nachvernetzer wird im Allgemeinen in einer Menge von mindestens 0,001 Gew.-%, vorzugsweise von mindestens 0,02 Gew.-%, in besonders bevorzugter Form von mindestens 0,05 Gew.% sowie im Allgemeinen höchstens 2 Gew.-%, vorzugsweise höchstens 1 Gew.-%, in besonders bevorzugter Form höchstens 0,3 Gew.-%, beispielsweise höchstens 0,15 Gew.-% oder höchstens 0,095 Gew.-% eingesetzt, jeweils auf die Masse des Grundpolymeren bezogen.

Die Nachvernetzung wird üblicherweise so durchgeführt, dass eine Lösung des Nachvernetzers auf die getrockneten Grundpolymerpartikel aufgesprüht wird. Im Anschluss an das Aufsprühen werden die mit Nachvernetzer beschichteten Polymerpartikel thermisch getrocknet, wobei die Nachvernetzungsreaktion sowohl vor als auch während der Trocknung stattfinden kann. Falls Oberflächennachvernetzer mit polymerisierbaren Gruppen verwendet werden, kann die Oberflächennachvernetzung auch durch radikalisch induzierte Polymerisation solcher Gruppen mittels gängiger Radikalbildner oder auch mittels energiereicher Strahlung wie beispielsweise UV-Licht erfolgen. Dies kann parallel oder anstatt der Verwendung von Nachvernetzern erfolgen, die kovalente oder ionische Bindungen zu funktionellen Gruppen an der Oberfläche der Grundpolymerpartikel ausbilden.

Das Aufsprühen der Nachvernetzerlösung wird vorzugsweise in Mischern mit bewegten Mischwerkzeugen, wie Schneckenmischern, Scheiben-, Paddel- oder Schaufelmischern oder Mischern mit anderen Mischwerkzeugen durchgeführt. Besonders bevorzugt sind jedoch Vertikalmischer. Es ist aber auch möglich die Nachvernetzerlösung in einem Wirbelbett aufzusprühen. Geeignete Mischer sind beispielsweise als Pflugschar^{®}-Mischer von Gebr. Lödige Maschinenbau GmbH, Elsener-Straße 7 - 9, 33102 Paderborn, Deutschland, oder als Schugi^{®} Flexomix^{®}-Mischer, Vrieco-Nauta^{®}-Mischer oder Turbulizer^{®}-Mischer von Hosokawa Micron BV, Gildenstraat 26, 7000 AB Doetinchem, Niederlande, erhältlich.

Die einsetzbaren Sprühdüsen unterliegen keiner Beschränkung. Geeignete Düsen und Zerstäubungssysteme sind beispielsweise in den folgenden Literaturstellen beschrieben: Zerstäuben von Flüssigkeiten, Expert-Verlag, Bd. 660, Reihe Kontakt & Studium, Thomas Richter (2004) sowie in Zerstäubungstechnik, Springer-Verlag, VDI-Reihe, Günter Wozniak (2002). Einsetzbar sind mono- und polydisperse Sprühsysteme. Unter den polydispersen Systemen sind Einstoff-Druckdüsen (strahl- oder lamellenbildend), Rotationszerstäuber, Zweistoffzerstäuber, Ultraschallzerstäuber und Pralldüsen geeignet. Bei den Zweistoffzerstäubern kann die Mischung der Flüssigkeits- mit der Gasphase sowohl innenliegend als auch außenliegend erfolgen. Das Sprühbild der Düsen ist unkritisch und kann jede beliebige Form annehmen, beispielsweise Rundstrahl-, Flachstrahl-, Weitwinkel-Rundstrahl- oder Kreisring-Spritzbild. Vorteilhaft ist die Verwendung eines nicht-oxidierenden Gases, falls Zweistoffzerstäuber eingesetzt werden, besonders bevorzugt sind Stickstoff, Argon oder Kohlendioxid. Derartigen Düsen kann die zu versprühende Flüssigkeit unter Druck zugeführt werden. Die Zerteilung der zu versprühenden Flüssigkeit kann dabei dadurch erfolgen, dass sie nach Erreichen einer bestimmten Mindestgeschwindigkeit in der Düsenbohrung entspannt wird. Ferner können für den erfindungsgemäßen Zweck auch Einstoffdüsen, wie beispielsweise Schlitzdüsen oder Drallkammern (Vollkegeldüsen) verwendet werden (beispielsweise von Düsen-Schlick GmbH, DE, oder von Spraying Systems Deutschland GmbH, DE). Derartige Düsen sind auch in der EP 0 534 228 A1 und der EP 1 191 051 A2 beschrieben.

Die Nachvernetzer werden typischerweise als wässrige Lösung eingesetzt. Wird ausschließlich Wasser als Lösungsmittel verwendet, so wird der Nachvernetzerlösung oder bereits dem Grundpolymer vorteilhafterweise ein Tensid oder Deagglomerisationshilfsmittel zugesetzt. Dadurch wird das Benetzungsverhalten verbessert und die Verklumpungsneigung vermindert.

Alle anionischen, kationischen, nichtionischen und amphoteren Tenside sind als Deagglomerationshilfsmittel geeignet, bevorzugt sind jedoch aus Hautverträglichkeitsgründen nicht-ionische und amphotere Tenside. Das Tensid kann auch Stickstoff enthalten. Beispielsweise werden Sorbitanmonoester, wie Sorbitanmonococoat und Sorbitanmonolaurat, oder ethoxylierte Varianten davon, wie beispielsweise Polysorbat 20^{®}, zugesetzt. Weitere geeignete Deagglomerationshilfsmittel stellen die ethoxylierten und alkoxylierten Derivate des 2-Propylheptanols dar, die unter den Marken Lutensol XL^{®} und Lutensol XP^{®} vertrieben werden (BASF SE, Carl-Bosch-Straße 38, 67056 Ludwigshafen, Deutschland).

Das Deagglomerationshilfsmittel kann getrennt dosiert oder der Nachvernetzerlösung zugesetzt werden. Vorzugsweise wird das Deagglomerationshilfsmittel der Nachvernetzerlösung einfach zugesetzt.

Die Einsatzmenge des Deagglomerationshilfsmittels bezogen auf Grundpolymer beträgt beispielsweise 0 bis 0,1 Gew.-%, vorzugsweise 0 bis 0,01 Gew.-%, besonders bevorzugt 0 bis 0,002 Gew.-%. Vorzugsweise wird das Deagglomerationshilfsmittel so dosiert, dass die Oberflächenspannung eines wässrigen Extrakts des gequollenen Grundpolymers und/oder des gequollenen nachvernetzten Superabsorbers bei 23 °C mindestens 0,060 N/m, vorzugsweise mindestens 0,062 N/m, besonders bevorzugt mindestens 0,065 N/m, und vorteilhaft höchstens 0,072 N/m beträgt.

Die wässrige Nachvernetzerlösung kann neben dem mindestens einen Nachvernetzer auch noch ein Cosolvens enthalten. Über den Gehalt an nichtwässrigem Lösungsmittel bzw. Gesamtlösungsmittelmenge kann die Eindringtiefe des Nachvernetzers in die Polymerpartikel eingestellt werden. Technisch gut geeignete Cosolventien sind C1-C6-Alkohole, wie Methanol, Ethanol, n-Propanol, Isopropanol, n-Butanol, sec-Butanol, tert.-Butanol oder 2-Methyl-1-propanol, C2-C5-Diole, wie Ethylenglykol, 1,2-Propylenglykol oder 1,4-Butandiol, Ketone, wie Aceton, oder Carbonsäureester, wie Essigsäureethylester. Nachteilig an einigen dieser Cosolventien ist, dass sie typische Eigengerüche aufweisen.

Das Cosolvens selbst ist unter den Reaktionsbedingungen idealerweise kein Nachvernetzer. Es kann jedoch im Grenzfall und abhängig von Verweilzeit und Temperatur dazu kommen, dass das Cosolvens teilweise zur Vernetzung beiträgt. Dies ist insbesondere dann der Fall, wenn der Nachvernetzer relativ träge ist und daher auch selbst sein Cosolvens bilden kann, wie beispielsweise bei Einsatz cyclischer Carbonate der allgemeinen Formel (IV), Diole der allgemeinen Formel (IIIa) oder Polyole der allgemeinen Formel (IIIb). Solche Nachvernetzer können im Gemisch mit reaktiveren Nachvernetzern auch in der Funktion als Cosolvens eingesetzt werden, da die eigentliche Nachvernetzungsreaktion dann bei niedrigeren Temperaturen und/oder kürzeren Verweilzeiten als in Abwesenheit des reaktiveren Vernetzers durchgeführt werden kann. Da das Cosolvens in relativ großen Mengen verwendet wird und auch teilweise im Produkt verbleibt, darf es nicht toxisch sein.

Im erfindungsgemäßen Verfahren eignen sich die Diole der allgemeinen Formel (IIa), die Polyole der allgemeinen Formel (IIb), sowie die cyclischen Carbonate der allgemeinen Formel (III) auch als Cosolventien. Diese Funktion erfüllen sie in Gegenwart eines reaktiven Nachvernetzers der allgemeinen Formel (I) und/oder (IV) und/oder einer Di - oder Triglycidylverbindung. Bevorzugte Cosolventien im erfindungsgemäßen Verfahren sind jedoch insbesondere die Diole der allgemeinen Formel (IIa), insbesondere, wenn die Hydroxygruppen sterisch durch Nachbargruppen an einer Reaktion behindert werden. Solche Diole eignen sich zwar prinzipiell auch als Nachvernetzer, erfordern dazu jedoch deutlich höhere Reaktionstemperaturen oder gegebenenfalls höhere Einsatzmengen als sterisch ungehinderte Diole.

Besonders bevorzugte Kombinationen aus wenig reaktivem Nachvernetzer als Cosolvens und reaktivem Nachvernetzer sind Kombinationen von bevorzugten mehrwertigen Alkoholen, Diolen der allgemeinen Formel (IIa) und Polyolen der allgemeinen Formel (IIb), mit Amidacetalen oder Carbamaten der allgemeinen Formel (I).

Geeignete Kombinationen sind beispielsweise 2-Oxazolidon/1,2-Propandiol und N-(2-Hydroxyethyl)-2-oxazolidon/1,2-Propandiol sowie Ethylenglykoldiglycidylether/1,2-Propandiol.

Ganz besonders bevorzugte Kombinationen sind 2-Oxazolidon/1,3-Propandiol und N-(2-Hydroxyethyl)-2-oxazolidon/1,3-Propandiol.

Weiterhin bevorzugte Kombinationen sind solche mit Ethylenglykoldiglycidylether oder Glyzerindi- oder -triglycidylether mit folgenden Lösemitteln, Cosolventien oder Covernetzern: Isopropanol, 1,3-Propandiol, 1,2-Propylenglykol oder Gemischen davon.

Weiterhin bevorzugte Kombinationen sind solche mit 2-Oxazolidon oder (2-Hydroxyethyl)-2-oxazolidon in folgenden Lösemitteln, Cosolventien oder Covernetzern: Isopropanol, 1,3-Propandiol, 1,2-Propylenglykol, Ethylencarbonat, Propylencarbonat oder Gemischen davon.

Häufig beträgt die Konzentration des Cosolvens in der wässrigen Nachvernetzerlösung, von 15 bis 50 Gew.-%, vorzugsweise von 15 bis 40 Gew.-%, besonders bevorzugt von 20 bis 35 Gew.-%, bezogen auf die Nachvernetzerlösung. Bei Cosolventien, die mit Wasser nur begrenzt mischbar sind, wird man vorteilhaft die wässrige Nachvernetzerlösung so einstellen, dass nur eine Phase vorliegt, gegebenenfalls durch Erniedrigung der Konzentration des Cosolvens.

In einer bevorzugten Ausführungsform wird kein Cosolvens eingesetzt. Der Nachvernetzer wird dann nur als Lösung in Wasser angewandt, gegebenenfalls unter Zusatz eines Deagglomerationshilfsmittels.

Die Konzentration des mindestens einen Nachvernetzers in der wässrigen Nachvernetzerlösung, beträgt typischerweise 1 bis 20 Gew.-%, vorzugsweise 1,5 bis 10 Gew.-%, besonders bevorzugt 2 bis 5 Gew.-%, bezogen auf die Nachvernetzerlösung.

Die Gesamtmenge der Nachvernetzerlösung bezogen auf Grundpolymer beträgt üblicherweise von 0,3 bis 15 Gew.-%, vorzugsweise von 2 bis 6 Gew.-%.

Die eigentliche Oberflächennachvernetzung durch Reaktion des Oberflächennachvernetzers mit funktionellen Gruppen an der Oberfläche der Grundpolymerpartikel wird meist durch Erwärmung des mit Oberflächennachvernetzerlösung benetzten Grundpolymers durchgeführt, üblicherweise "Trocknung" genannt (aber nicht mit der oben beschriebenen Trocknung des Polymergels aus der Polymerisation zu verwechseln, bei der typischerweise sehr viel mehr Flüssigkeit zu entfernen ist). Die Trocknung kann im Mischer selbst erfolgen, durch Beheizung des Mantels, durch Wärmeaustauschflächen oder Einblasen warmer Gase. Gleichzeitiges Versetzen des Superabsorbers mit Oberflächennachvernetzer und Trocknen kann beispielsweise in einem Wirbelschichttrockner erfolgen. Die Trocknung wird aber meist in einem nachgeschalteten Trockner, wie beispielsweise einem Hordentrockner, einem Drehrohrofen, ein Paddel- oder Scheibentrockner oder einer beheizbaren Schnecke durchgeführt. Geeignete Trockner sind beispielsweise als Solidair^{®} oder Torusdisc^{®}-Trockner von Bepex International LLC, 333 N.E. Taft Street, Minneapolis, MN 55413, U.S.A., oder als Paddel- oder Schaufeltrockner oder auch als Fließbetttrockner von Nara Machinery Co., Ltd., Zweigniederlassung Europa, Europaallee 46, 50226 Frechen, Deutschland erhältlich.

Es ist möglich die Polymerpartikel zur Trocknung und Durchführung der Oberflächennachvernetzung über Kontaktflächen in einem nachgeschalteten Trockner zu beheizen, oder über zugeführtes warmes Inertgas, oder über eine Mischung eines oder mehrerer Inertgase mit Wasserdampf, oder nur mit Wasserdampf allein. Bei Zufuhr der Wärme über Kontaktflächen ist es möglich die Reaktion bei leichtem oder vollständigem Unterdruck unter Inertgas durchzuführen. Bei Verwendung von Wasserdampf zum direkten Beheizen der Polymerpartikel ist es erfindungsgemäß wünschenswert den Trockner bei Normaldruck oder Überdruck zu betreiben. In diesem Fall kann es sinnvoll sein den Nachvernetzungsschritt in einen Aufheizschritt mit Wasserdampf und einen Reaktionsschritt unter Inertgas aber ohne Wasserdampf aufzuspalten. Dies kann in einem oder mehreren Apparaten realisiert werden. Erfindungsgemäß können die Polymerpartikel schon im Nachvernetzungsmischer mit Wasserdampf aufgeheizt werden. Das eingesetzte Grundpolymer kann aus vorhergehenden Prozessschritten noch eine Temperatur von 10 bis 120 °C aufweisen, die Nachvernetzerlösung kann eine Temperatur von 0 bis 70 °C aufweisen. Insbesondere kann die Nachvernetzerlösung zur Verminderung der Viskosität erwärmt werden.

Bevorzugte Trocknungstemperaturen liegen im Bereich 100 bis 250°C, bevorzugt 120 bis 220°C, besonders bevorzugt 130 bis 210°C, ganz besonders bevorzugt 150 bis 200°C. Die bevorzugte Verweilzeit bei dieser Temperatur im Reaktionsmischer oder Trockner beträgt vorzugsweise mindestens 10 Minuten, besonders bevorzugt mindestens 20 Minuten, ganz besonders bevorzugt mindestens 30 Minuten, und üblicherweise höchstens 60 Minuten. Typischerweise wird die Trocknung so geführt, dass der Superabsorber einen Restfeuchtegehalt von im Allgemeinen mindestens 0,1 Gew.-%, vorzugsweise mindestens 0,2 Gew.-% und in besonders bevorzugter Form mindestens 0,5 Gew.-% sowie im Allgemeinen höchstens 15 Gew.-%, vorzugsweise höchstens 10 Gew.-% und in besonders bevorzugter Form höchstens 8 Gew.-% aufweist.

Die Nachvernetzung kann unter normalen atmosphärischen Bedingungen stattfinden. Normale atmosphärische Bedingungen bedeutet, dass keine technischen Vorkehrungen getroffen werden, um den Partialdruck oxidierender Gase wie den des atmosphärischen Sauerstoffs im Apparat, in dem die Nachvernetzungsreaktion überwiegend stattfindet (dem "Nachvernetzungsreaktor", typischerweise der Trockner) zu verringern. Es ist jedoch bevorzugt, die Nachvernetzungsreaktion unter verringertem Partialdruck oxidierender Gase durchzuführen. Oxidierende Gase sind Stoffe, die bei 23 °C einen Dampfdruck von mindestens 1013 mbar aufweisen und in Verbrennungsvorgängen als Oxidationsmittel wirken, beispielsweise Sauerstoff, Stickoxid und Stickstoffdioxid, insbesondere Sauerstoff. Vorzugsweise beträgt der Partialdruck oxidierender Gase dabei weniger als 140 mbar, bevorzugt weniger als 100 mbar, besonders bevorzugt weniger als 50 mbar, ganz besonders bevorzugt weniger als 10 mbar. Wird die thermische Nachvernetzung bei Umgebungsdruck, d. h. bei einem Gesamtdruck um 1013 mbar, durchgeführt, so wird der Gesamtpartialdruck der oxidierenden Gase über deren Volumenanteil festgelegt. Der Anteil der oxidierenden Gase beträgt dabei vorzugsweise weniger als 14 Vol.-%, bevorzugt weniger als 10 Vol.-%, besonders bevorzugt weniger als 5 Vol.-%, ganz besonders bevorzugt weniger als 1 Vol.-%.

Die Nachvernetzung kann unter vermindertem Druck durchgeführt werden, d. h. bei einem Gesamtdruck von weniger als 1.013 mbar. Der Gesamtdruck beträgt typischerweise weniger als 670 mbar, vorzugsweise weniger als 480 mbar, besonders bevorzugt weniger als 300 mbar, ganz besonders bevorzugt weniger als 200 mbar. Werden Trocknung und Nachvernetzung unter Luft mit einem Sauerstoffgehalt von 20,8 Vol.-% durchgeführt, so betragen die zu den obengenannten Gesamtdrücken korrespondierenden Sauerstoffpartialdrücke 139 mbar (670 mbar), 100 mbar (480 mbar), 62 mbar (300 mbar) und 42 mbar (200 mbar), wobei die jeweiligen Gesamtdrücke in den Klammern stehen. Eine andere Möglichkeit, den Partialdruck oxidierender Gase zu senken, ist die Einleitung von nicht oxidierenden Gasen, insbesondere Inertgasen in den zur Nachvernetzung verwendeten Apparat. Geeignete Inertgase sind bei der Nachvernetzungstemperatur und gegebenem Druck im Nachvernetzungstrockner gasförmig vorliegende Stoffe, die unter diesen Bedingungen nicht oxidierend auf die Bestandteile der trocknenden Polymerpartikel wirken, beispielsweise Stickstoff, Kohlendioxid, Argon, Wasserdampf, wobei Stickstoff bevorzugt ist. Die Inertgasmenge beträgt im Allgemeinen von 0,0001 bis 10 m³, bevorzugt 0,001 bis 5 m³, besonders bevorzugt 0,005 bis 1 m³, und ganz besonders bevorzugt 0,005 bis 0,1 m³, bezogen auf 1 kg Superabsorber.

Im erfindungsgemäßen Verfahren kann das Inertgas, wenn es nicht Wasserdampf enthält, über Düsen in den Nachvernetzungstrockner eingeblasen werden, besonders bevorzugt wird das Inertgas aber bereits im oder kurz vor dem Mischer, indem der Superabsorber mit Oberflächennachvernetzer versetzt wird, über Düsen dem Polymerpartikelstrom zugegeben.

Selbstverständlich können aus dem Trockner abgeführte Dämpfe von Cosolventien außerhalb des Trockners wieder kondensiert und gegebenenfalls rezykliert werden.

In einer bevorzugten Ausführungsform der vorliegenden Erfindung werden vor, während oder nach der Nachvernetzung zusätzlich zu den Nachvernetzern polyvalente Kationen auf die Partikeloberfläche aufgebracht. Dies ist im Prinzip eine weitere Oberflächennachvernetzung durch ionische, nicht kovalente Bindungen, wird aber gelegentlich auch als "Komplexierung" mit den betreffenden Metallionen oder einfach als "Beschichtung" mit den betreffenden Substanzen (dem "Komplexierungsmittel") bezeichnet.

Dieses Aufbringen von polyvalenten Kationen erfolgt durch Aufsprühen von Lösungen zwei- oder mehrwertiger Kationen, meist zwei-, drei- oder vierwertiger Metallkationen, aber auch polyvalenter Kationen wie formal ganz oder teilweise aus Vinylaminmonomeren aufgebauter Polymere wie teilweise oder vollständig hydrolysiertes Polyvinylamid (sogenanntes "Polyvinylamin"), dessen Amingruppen stets - auch bei sehr hohen pH-Werten - teilweise zu Ammoniumgruppen protoniert vorliegen. Beispiele verwendbarer zweiwertiger Metallkationen sind insbesondere die zweiwertigen Kationen von Metallen der Gruppen 2 (insbesondere Mg, Ca, Sr, Ba), 7 (insbesondere Mn), 8 (insbesondere Fe), 9 (insbesondere Co), 10 (insbesondere Ni), 11 (insbesondere Cu) und 12 (insbesondere Zn) des Periodensystems der Elemente. Beispiele verwendbarer dreiwertiger Metallkationen sind insbesondere die dreiwertigen Kationen von Metallen der Gruppen 3 einschließlich der Lanthaniden (insbesondere Sc, Y, La, Ce), 8 (insbesondere Fe), 11 (insbesondere Au), 13 (insbesondere Al) und 14 (insbesondere Bi) des Periodensystems der Elemente. Beispiele verwendbarer vierwertiger Kationen sind insbesondere die vierwertigen Kationen von Metallen der Lanthaniden (insbesondere Ce) sowie der Gruppe 4 (insbesondere Ti, Zr, Hf) des Periodensystems der Elemente. Die Metallkationen können sowohl allein als auch im Gemisch untereinander eingesetzt werden. Besonders bevorzugt ist die Verwendung dreiwertiger Metallkationen. Ganz besonders bevorzugt ist die Verwendung von Aluminiumkationen.

Von den genannten Metall-Kationen sind alle Metallsalze geeignet, die eine ausreichende Löslichkeit in dem zu verwendenden Lösungsmittel besitzen. Besonders geeignet sind Metallsalze mit schwach komplexierenden Anionen wie zum Beispiel Chlorid, Nitrat und Sulfat, Hydrogensulfat, Carbonat, Hydrogencarbonat, Nitrat, Phosphat, Hydrogenphosphat, oder Dihydrogenphosphat. Bevorzugt sind Salze von Mono- und Dicarbonsäuren, Hydroxisäuren, Ketosäuren sowie Aminosäuren oder basische Salze. Beispielsweise genannt seien bevorzugt Acetate, Propionate, Tartrate, Maleate, Citrate, Laktate, Malate, Succinate. Ebenso bevorzugt ist die Verwendung von Hydroxiden. Besonders bevorzugt ist die Verwendung von 2-Hydroxicarbonsäuresalzen wie Citraten und Laktaten. Beispiele besonders bevorzugter Metallsalze sind Alkali- und Erdalkalimetallaluminate und deren Hydrate, etwa Natriumaluminat und dessen Hydrate, Alkali- und Erdalkalimetalllactate und -citrate und deren Hydrate, Aluminiumacetat, Aluminiumpropionat, Aluminiumcitrat und Aluminiumlaktat.

Die genannten Kationen und Salze können in Reinform oder als Gemisch verschiedener Kationen oder Salze verwendet werden. Die eingesetzten Salze des zwei und/oder dreiwertigen Metallkations können weitere Nebenbestandteile wie noch unneutralisierte Carbonsäure und/oder Alkalisalze der neutralisierten Carbonsäure enthalten. Bevorzugte Alkalisalze sind die des Natriums, Kaliums und des Ammoniums. Sie werden typischerweise als wässerige Lösung eingesetzt, welche durch Auflösen der festen Salze in Wasser gewonnen wird, oder bevorzugt direkt als solche erzeugt wird, wodurch gegebenenfalls Trocknungs- und Reinigungsschritte vermieden werden. Vorteilhaft können auch die Hydrate der genannten Salze eingesetzt werden, die sich oft schneller in Wasser lösen als die wasserfreien Salze.

Die Einsatzmenge an Metallsalz beträgt im Allgemeinen mindestens 0,001 Gew.-%, vorzugsweise mindestens 0,01 Gew.-% und in besonders bevorzugter Form mindestens 0,1 Gew.-%, beispielsweise mindestens 0,4 Gew.-% sowie im Allgemeinen höchstens 5 Gew.-%, vorzugsweise höchstens 2,5 Gew.-% und in besonders bevorzugter Form höchstens 1 Gew.-%, beispielsweise höchstens 0,7 Gew.-% jeweils bezogen auf die Masse des Grundpolymeren.

Das Salz des dreiwertigen Metallkations kann als Lösung oder Suspension eingesetzt werden. Als Lösungsmittel für die Metallsalze können Wasser, Alkohole, DMF, DMSO sowie Mischungen dieser Komponenten eingesetzt werden. Besonders bevorzugt sind Wasser und Wasser/Alkohol-Mischungen wie zum Bespiel Wasser/Methanol, Wasser/1,2-Propandiol und Wasser/1,3-Propandiol.

Die Behandlung des Grundpolymeren mit Lösung eines zwei- oder mehrwertigen Kations erfolgt in gleicher Weise wie die mit Oberflächennachvernetzer, einschließlich des Trocknungsschritts. Oberflächennachvernetzer und polyvalentes Kation können in einer gemeinsamen Lösung oder als getrennte Lösungen aufgesprüht werden. Das Aufsprühen der Metallsalz-Lösung auf die Superabsorberpartikel kann sowohl vor als auch nach der Oberflächennachvernetzung erfolgen. In einem besonders bevorzugten Verfahren erfolgt die Aufsprühung der Metallsalz-Lösung im gleichen Schritt mit dem Aufsprühen der Vernetzer-Lösung, wobei beide Lösungen getrennt nacheinander oder gleichzeitig über zwei Düsen aufgesprüht werden, oder Vernetzer- und Metallsalz-Lösung vereint über eine Düse aufgesprüht werden können.

Insbesondere wenn ein drei- oder höhervalentes Metallkation wie Aluminium zur Komplexierung verwendet wird, wird wahlweise auch ein basisches Salz eines zweiwertigen Metallkations oder ein Gemisch solcher Salze zugegeben. Basische Salze sind Salze, die geeignet sind den pH-Wert einer sauren wässrigen Lösung zu erhöhen, vorzugsweise einer 0,1 N Salzsäure. Basische Salze sind üblicherweise Salze einer starken Base mit einer schwachen Säure.

Das zweiwertige Metallkation des optionalen basischen Salzes ist vorzugsweise ein Metallkation der Gruppe 2 des Periodensystems der Elemente, besonders bevorzugt Calcium oder Strontium, ganz besonders bevorzugt Calcium.

Die basischen Salze der zweiwertigen Metallkationen sind vorzugsweise Salze schwacher anorganischer Säuren, schwacher organischer Säuren und/oder Salze von Aminosäuren, besonders bevorzugt Hydroxide, Hydrogencarbonate, Carbonate, Acetate, Propionate, Citrate, Glukonate, Laktate, Tartrate, Malate, Succinate, Maleate und/oder Fumarate, ganz besonders bevorzugt Hydroxide, Hydrogencarbonate, Carbonate, Propionate und/oder Laktate. Das basische Salz ist vorzugsweise wasserlöslich. Wasserlösliche Salze sind Salze, welche bei 20 °C eine Wasserlöslichkeit von mindestens 0,5 g Salz pro Liter Wasser, vorzugsweise mindestens 1 g Salz pro I Wasser, bevorzugt mindestens 10 g Salz pro I Wasser, besonders bevorzugt mindestens 100 g Salz pro I Wasser, ganz besonders bevorzugt mindestens 200 g Salz pro I Wasser, aufweisen. Erfindungsgemäß verwendbar sind jedoch auch solche Salze, die diese Mindestlöslichkeit bei der Aufsprühtemperatur der Sprühlösung aufweisen. Vorteilhaft können auch die Hydrate der genannten Salze eingesetzt werden, die sich oft schneller in Wasser lösen als die wasserfreien Salze.

Geeignete basische Salze zweiwertiger Metallkationen sind beispielsweise Calciumhydroxid, Strontiumhydroxid, Calciumhydrogencarbonat, Strontiumhydrogencarbonat, Calciumacetat, Strontiumacetat, Calciumpropionat, Calciumlaktat, Strontiumpropionat, Strontiumlaktat, Zinklaktat, Calciumcarbonat und Strontiumcarbonat.

Wenn die Wasserlöslichkeit nicht ausreicht eine Sprühlösung der gewünschten Konzentration herzustellen, dann können auch Dispersionen des festen Salzes in seiner gesättigten wässerigen Lösung eingesetzt werden. Beispielsweise können Calciumcarbonat, Strontiumcarbonat, Calciumsulfit, Strontiumsulfit, Calciumphosphat und Strontiumphosphat auch als wässrige Dispersionen eingesetzt werden.

Die Menge an basischem Salz des zweiwertigen Metallkations, bezogen auf die Masse des Grundpolymers, beträgt typischerweise von 0,001 bis 5 Gew.-%, vorzugsweise von 0,01 bis 2,5 Gew.-%, bevorzugt 0,1 bis 1,5 Gew.-%, besonders bevorzugt von 0,1 bis 1 Gew.-%, ganz besonders bevorzugt von 0,4 bis 0,7 Gew.-%.

Das basische Salz des zweiwertigen Metallkations kann als Lösung oder Suspension eingesetzt werden. Beispiele hierfür sind Calciumlaktat-Lösungen oder Calciumhydroxid-Suspensionen. Üblicherweise werden die Salze mit einer Wassermenge von nicht mehr als 15 Gew.-% vorzugsweise von nicht mehr als 8 Gew.-%, besonders bevorzugt von nicht mehr als 5 Gew.-%, ganz besonders bevorzugt von nicht mehr als 2 Gew.-% bezogen auf den Superabsorber aufgesprüht.

Vorzugsweise wird eine wässrige Lösung des basischen Salzes auf den Superabsorber aufgesprüht. Bequemerweise wird das basische Salz gleichzeitig mit dem Oberflächennachvernetzungsmittel, dem Komplexierungsmittel oder als weiterer Bestandteil der Lösungen dieser Mittel zugegeben. Für diese basischen Salze ist die Zugabe im Gemisch mit dem Komplexierungsmittel bevorzugt. Wenn die Lösung des basischen Salzes nicht mit der Lösung des Komplexierungsmittels ohne Ausfällung mischbar ist, so können die Lösungen separat nacheinander oder zeitgleich aus zwei Düsen aufgesprüht werden.

Dem Superabsorber wird wahlweise auch eine reduzierende Verbindung zugesetzt. Beispiele reduzierender Verbindungen sind Hypophosphite, Sulfinate oder Sulfite. Bevorzugt ist der Zusatz eines Sulfinsäure-Derivats, insbesondere einer Verbindung der Formel (V) 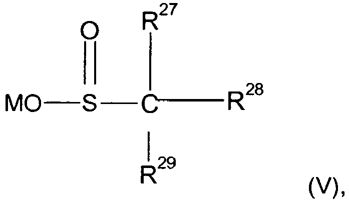 worin
- M: für ein Wasserstoffatom, ein Ammoniumion, ein einwertiges Metallion oder ein Äquivalent eines zweiwertigen Metallions der Gruppen 1, 2, 8, 9, 10, 12 oder 14 des Periodensystems der Elemente steht;
- R²⁷: für OH oder NR³⁰R³¹ steht, wobei R³⁰ und R³¹ unabhängig voneinander für H oder C₁-C₆-Alkyl stehen;
- R²⁸: für H oder eine Alkyl-, Alkenyl-, Cycloalkyl- oder Arylgruppe steht, wobei diese Gruppe wahlweise 1, 2 oder 3 Substituenten aufweist, die unabhängig voneinander ausgewählt sind unter C₁-C₆-Alkyl, OH, O-C₁-C₆-Alkyl, Halogen und CF₃; und
- R²⁹: für COOM, SO₃M, COR³⁰,CONR³⁰R³¹ oder COOR³⁰ steht, wobei M, R³⁰ und R³¹ die oben angegebenen Bedeutungen besitzen oder, wenn R²⁸ für Aryl steht, das wahlweise wie oben angegeben substituiert ist, auch für H steht,
Salzen davon oder Gemischen solcher Verbindungen und/oder Salzen davon.

In obiger Formel steht Alkyl für geradkettige oder verzweigte Alkylgruppen, die vorzugsweise 1 - 6, insbesondere 1 - 4 Kohlenstoffatome aufweisen. Beispiele für Alkylgruppen sind Methyl, Ethyl, n-Propyl, Isopropyl, n-Butyl, t-Butyl, n-Hexyl etc. Entsprechendes gilt für die Alkylgruppen in O-Alkyl. Alkenyl steht für geradkettige oder verzweigte Alkenylgruppen, die vorzugsweise 3 - 8 Kohlenstoffatome, insbesondere 3 - 6 Kohlenstoffatome aufweisen. Eine bevorzugte Alkenylgruppe ist die Allylgruppe. Cycloalkyl steht insbesondere für C₁-C₆-Cycloalkyl, wobei Cyclopentyl und Cyclohexyl besonders bevorzugt sind. Aryl (auch in Aralkyl) steht vorzugsweise für Phenyl oder Naphthyl. Wenn der Arylrest für eine Phenylgruppe steht und substituiert ist, so weist er vorzugsweise zwei Substituenten auf. Diese sind insbesondere in 2- und/oder 4-Stellung vorhanden.

Halogen steht für F, Cl, Br und I, vorzugsweise für Cl und Br.

M steht vorzugsweise für ein Ammonium-, Alkalimetall- oder ein Äquivalent eines Erdalkalimetall- oder Zinkions. Geeignete Alkalimetallionen sind insbesondere Natrium - und Kaliumionen, geeignete Erdalkalimetallionen sind vor allem Magnesium-, Strontium- und Calciumionen.

R²⁷ steht vorzugsweise für eine Hydroxi- oder Aminogruppe.
R²⁸ steht vorzugsweise für ein Wasserstoffatom oder eine Alkyl- oder Arylgruppe, die wie oben substituiert sein kann. Vorzugsweise weist sie einen oder zwei Hydroxi - und/oder Alkoxisubstituenten auf.

R²⁹ steht vorzugsweise entweder für COOM oder COOR³⁰ (M und R³⁰ besitzen die oben angegebenen Bedeutungen) oder, wenn R²⁷ für Aryl steht, das wie oben angegeben substituiert sein kann, auch für ein Wasserstoffatom.

In einer bevorzugten Ausführungsform werden dem Superabsorber Verbindungen der obigen Formel (V) zugesetzt, worin M für ein Alkalimetallion oder ein Äquivalent eines Erdalkalimetall- oder Zinkions steht; R²⁷ für eine Hydroxi- oder Aminogruppe steht; R²⁸ für H oder Alkyl steht und R²⁹ für COOM oder COOR³⁰ steht, wobei, wenn R²⁹ für COOM steht, M in diesem COOM-Rest für H, ein Alkalimetallion oder ein Äquivalent eines Erdalkalimetallions steht und wenn R²⁹ für COOR³⁰ steht, R³⁰ für C₁-C₆-Alkyl steht.

In einer weiteren bevorzugten Ausführungsform werden dem Superabsorber Verbindungen der obigen Formel (V) zugesetzt, worin M für ein Alkalimetallion oder ein Äquivalent eines Erdalkalimetall- oder Zinkions steht; R²⁷ für eine Hydroxi- oder Aminogruppe steht; R²⁸ für Aryl, das wahlweise wie oben angegeben substituiert ist, insbesondere für Hydroxiphenyl oder C₁-C₄-Alkoxyphenyl steht; und R²⁹ für ein Wasserstoffatom steht.

Die Gruppen 1 (H, Li, Na, K, Rb, Cs, Fr), 2 (Be, Mg, Ca, Sr, Ba, Ra), 8 (Fe, Ru, Os), 9 (Co, Rh, Ir), 10 (Ni, Pd, Pt), 12 (Zn, Cd, Hg) und 14 (C, Si, Ge, Sn, Pb) des Periodensystems der Elemente in der aktuellen Nummerierung der IUPAC (International Union of Pure and Applied Chemistry, 104 T.W. Alexander Drive, Building 19, Research Triangle Park, NC 27709, U.S.A., www.iupac.org), der für Nomenklatur im Bereich der Chemie zuständigen internationalen Organisation, entsprechen den Gruppen la, IIa, IIb, IVa und VIIIb in der von CAS (Chemical Abstracts Service, 2540 Olentangy River Road, Columbus, OH 43202, U.S.A., www.cas.org) verwendeten Nummerierung.

Die Sulfinsäurederivate der obigen Formel (V) können in Reinform zugesetzt werden, wahlweise aber auch in dem aus der Herstellung solcher Verbindungen auf übliche Weise resultierenden Gemisch mit dem Sulfit des entsprechenden Metallions und der entsprechenden Sulfonsäure. Die Herstellung derartiger Sulfinsäurederivate der obigen Formel ist bekannt und beispielsweise in WO 99/18 067 A1 beschrieben. Sie sind auch gängige Handelswaren und beispielsweise in der Form von Gemischen aus dem Natriumsalz der 2-Hydroxy-2-sulfinatoessigsäure, dem Dinatriumsalz der 2-Hydroxy-2-sulfonatoessigsäure und Natriumbisulfit von L. Brüggemann KG (Salzstraße 131, 74076 Heilbronn, Deutschland, www.brueggemann.com) unter den Bezeichnungen BRÜGGOLIT^{®} FF6M oder BRÜGGOLIT^{®} FF7, alternativ BRUGGOLITE^{®} FF6M oder BRUGGOLITE^{®} FF7 erhältlich.

Der Zusatz einer oder mehrerer reduzierender Verbindungen zum Superabsorber erfolgt auf übliche Weise durch Zugabe der Verbindung in Substanz, als Lösung oder als Suspension in einem Lösungs- oder Suspensionsmittel während oder nach der Herstellung des Superabsorbers. Typischerweise wird eine Lösung oder Suspension der reduzierenden Verbindung in Wasser oder einem organischen Lösungsmittel verwendet, beispielsweise in einem Alkohol oder Polyol oder in Mischungen davon. Beispiele geeigneter Lösungs- oder Suspensionsmittel sind Wasser, Isopropanol/Wasser, 1,3-Propandiol/Wasser und Propylenglykol/Wasser, wobei das Mischungsmassenverhältnis vorzugsweise von 20:80 bis 40:60 beträgt. Der Lösung oder Suspension kann ein Tensid zugesetzt werden. Falls reduzierende Verbindungen zugesetzt werden, werden sie im Allgemeinen in einer Menge von mindestens 0,0001 Gew.-%, vorzugsweise mindestens 0,001 Gew.-% und in besonders bevorzugter Form mindestens 0,025 Gew.-%, beispielsweise mindestens 0,1 Gew.-% oder mindestens 0,3 Gew.-% sowie im Allgemeinen höchstens 3 Gew.-%, in bevorzugter Form höchstens 2,5 Gew.-% und in besonders bevorzugter Form höchstens 1,5 Gew.-%, beispielsweise höchstens 1 Gew.-% oder 0,7 Gew.-% zugesetzt, jeweils auf das Gesamtgewicht des Superabsorbers bezogen.

Die reduzierende Verbindung wird im Allgemeinen auf genau die gleiche Weise mit dem an sich bekannten Superabsorber vermischt wie die zur Oberflächennachvernetzung auf den Superabsorber aufgebrachte, einen Oberflächennachvernetzer enthaltende Lösung oder Suspension. Die reduzierende Verbindung kann als Bestandteil der zur Oberflächennachvernetzung aufgebrachten Lösung oder einer ihrer Komponenten auf ein Grundpolymer aufgebracht werden, also der Lösung des Oberflächennachvernetzers oder einer ihrer Komponenten zugesetzt werden. Der mit Oberflächennachvernetzungsfnittel und reduzierender Verbindung beschichtete Superabsorber durchläuft dann die weiteren zur Oberflächennachvernetzung erforderlichen Verfahrensschritte, beispielsweise eine thermisch induzierte Reaktion des Oberflächennachvernetzungsmittels mit dem Superabsorber. Dieses Verfahren ist vergleichsweise einfach und wirtschaftlich.

Falls höchste Stabilität gegen Verfärbung bei längerer Lagerung wesentlich ist, wird die reduzierende Verbindung vorzugsweise nach der Oberflächennachvernetzung in einem eigenen Verfahrensschritt aufgebracht. Falls sie als Lösung oder Suspension aufgebracht wird, erfolgt die Aufbringung dabei auf den bereits oberflächennachvernetzten Superabsorber in gleicher Weise wie die Aufbringung des Oberflächennachvernetzungsmittels auf das Grundpolymer. Meist, aber nicht notwendigerweise wird anschließend ebenso wie bei der Oberflächennachvernetzung erwärmt, um den Superabsorber wieder zu trocknen. Die bei dieser Trocknung eingestellte Temperatur liegt dann aber im Allgemeinen bei höchstens 110 °C, vorzugsweise höchstens 100 °C und in besonders bevorzugter Weise höchstens 90 °C, um unerwünschte Reaktionen der reduzierenden Verbindung zu vermeiden. Die Temperatur wird so eingestellt, dass in Anbetracht der Verweilzeit im Trocknungsaggregat der gewünschte Wassergehalt des Superabsorbers erreicht wird. Es ist durchaus auch möglich und bequem, die reduzierende Verbindung einzeln oder gemeinsam mit anderen üblichen Hilfsmitteln, beispielsweise Staubbindemitteln, Mitteln gegen Verbackung oder Wasser zur Rückbefeuchtung des Superabsorbers, wie unten für diese Hilfsmittel beschrieben zuzugeben, beispielsweise in einem der Oberflächennachvernetzung nachgeschalteten Kühler. Die Temperatur der Polymerpartikel beträgt in diesem Fall zwischen 0 °C und 190 °C, bevorzugt weniger als 160 °C, mehr bevorzugt weniger als 130 °C, noch mehr bevorzugt weniger als 100 °C, und am meisten bevorzugt weniger als 70 °C. Die Polymerpartikel werden gegebenenfalls nach Beschichtung zügig auf Temperaturen unterhalb der Zersetzungstemperatur der reduzierenden Verbindung abgekühlt.

Sofern im Anschluss an die Oberflächennachvernetzung und/oder Behandlung mit Komplexbildner ein Trocknungsschritt durchgeführt wird, ist es vorteilhaft, aber nicht unbedingt notwendig, das Produkt nach der Trocknung zu kühlen. Die Kühlung kann kontinuierlich oder diskontinuierlich erfolgen, bequemerweise wird das Produkt dazu kontinuierlich in einen dem Trockner nachgeschalteten Kühler gefördert. Dazu kann jeder zur Abfuhr von Wärme aus pulverförmigen Feststoffen bekannte Apparat verwendet werden, insbesondere jede oben als Trocknungsapparat erwähnte Vorrichtung, sofern sie nicht mit einem Heizmedium, sondern mit einem Kühlmedium wie etwa mit Kühlwasser beaufschlagt wird, so dass über die Wände und je nach Konstruktion auch über die Rührorgane oder sonstige Wärmeaustauschflächen keine Wärme in den Superabsorber eingetragen, sondern daraus abgeführt wird. Bevorzugt ist die Verwendung von Kühlern, in denen das Produkt bewegt wird, also gekühlten Mischern. beispielsweise Schaufelkühlern, Scheibenkühlern oder Paddelkühlern. Der Superabsorber kann auch in der Wirbelschicht durch Einblasen eines gekühlten Gases wie kalter Luft gekühlt werden. Die Bedingungen der Kühlung werden so eingestellt, dass ein Superabsorber mit der für die Weiterverarbeitung gewünschten Temperatur erhalten wird. Typischerweise wird eine mittlere Verweilzeit im Kühler von im Allgemeinen mindestens 1 Minute, vorzugsweise mindestens 3 Minuten und in besonders bevorzugter Form mindestens 5 Minuten sowie im Allgemeinen höchstens 6 Stunden, vorzugsweise höchstens 2 Stunden und in besonders bevorzugter Weise höchstens 1 Stunde eingestellt und die Kühlleistung so bemessen, dass das erhaltene Produkt eine Temperatur von im Allgemeinen mindestens 0 °C, vorzugsweise mindestens 10 °C und in besonders bevorzugter Form mindestens 20 °C sowie im Allgemeinen höchstens 100 °C, vorzugsweise höchstens 80 °C und in besonders bevorzugter Form höchstens 60 °C aufweist.

Der oberflächennachvernetzte Superabsorber wird wahlweise in üblicher Weise gemahlen und/oder gesiebt. Mahlung ist hier typischerweise nicht erforderlich, meist ist aber zur Einstellung der gewünschten Partikelgrößenverteilung des Produkts das Absieben von gebildeten Agglomeraten oder Feinkorn angebracht. Agglomerate und Feinkorn werden entweder verworfen oder vorzugsweise in bekannter Weise und an geeigneter Stelle in das Verfahren zurückgeführt; Agglomerate nach Zerkleinerung. Die für oberflächennachvernetzte Superabsorbern gewünschten Partikelgrößen sind die gleichen wie bei Grundpolymeren.

Wahlweise können auf die Oberfläche der Superabsorberpartikel, ob nicht nachvernetzt oder nachvernetzt, im Herstellverfahren in jedem Prozessschritt bei Bedarf alle bekannten Beschichtungen, wie filmbildende Polymere, thermoplastische Polymere, Dendrimere, polykationische Polymere (wie beispielsweise Polyvinylamin, Polyethylenimin oder Polyallylamin), wasserunlösliche polyvalente Metallsalze, wie beispielsweise Magnesiumcarbonat, Magnesiumoxid, Magnesiumhydroxid, Calciumcarbonat, Calciumsulfat oder Calciumphosphat, alle dem Fachmann bekannten wasserlöslichen mono- oder polyvalenten Metallsalze, wie beispielsweise Aluminiumsulfat, Natrium-, Kalium-, Zirkonium- oder Eisensalze, oder hydrophile anorganische Partikel, wie Tonminerale, pyrogene Kieselsäure, kolloidale Kieselsäuresole wie z.B. Levasil^{®}, Titandioxid, Aluminiumoxid und Magnesiumoxid, zusätzlich aufgebracht werden. Beispiele für nützliche Alkalimetallsalze sind Natrium- und Kaliumsulfat, Natrium- und Kaliumlaktate, - citrate, -sorbate. Dadurch können zusätzliche Effekte, beispielsweise eine verringerte Verbackungsneigung des Endprodukts oder des Zwischenprodukts im jeweiligen Prozessschritt des Herstellverfahrens, verbesserte Verarbeitungseigenschaften oder eine weiter gesteigerte Flüssigkeitsleitfähigkeit (SFC) erreicht werden. Wenn die Additive in Form von Dispersionen eingesetzt und aufgesprüht werden, dann werden sie bevorzugt als wässrige Dispersionen eingesetzt, und es wird bevorzugt noch zusätzlich ein Entstaubungsmittel zur Fixierung des Additivs auf der Oberfläche des Superabsorbers aufgebracht. Das Entstaubungsmittel wird dann entweder direkt der Dispersion des anorganischen pulvrigen Additivs hinzugefügt, optional kann es auch als separate Lösung vor, während, oder nach dem Auftrag des anorganischen pulvrigen Additivs durch Aufsprühen hinzugefügt werden. Am meisten bevorzugt ist die gleichzeitige Aufsprühung von Nachvernetzungsmittel, Entstaubungsmittel und pulvrigem anorganischen Additiv in der Nachvernetzung. In einer weiteren bevorzugten Verfahrensvariante wird das Entstaubungsmittel aber separat im Kühler zugegeben, beispielsweise durch Aufsprühen von oben, unten oder von der Seite. Besonders geeignete Entstaubungsmittel, die auch zur Fixierung pulvriger anorganischer Additive an der Oberfläche der Superabsorberpartikel dienen können, sind Polyethylenglykole mit einem Molekulargewicht von 400 bis 20000 g/mol, Polyglyzerin, 3- bis 100-fach ethoxylierte Polyole, wie Trimethylolpropan, Glyzerin, Sorbitol und Neopentylglykol. Besonders geeignet sind 7- bis 20-fach ethoxyliertes Glyzerin oder Trimethylolpropan, wie beispielsweise Polyol TP 70^{®} (Perstorp, SE). Letztere haben insbesondere den Vorteil, dass sie die Oberflächenspannung eines wässrigen Extrakts der Superabsorberpartikel nur unwesentlich herabsetzen.

Es ist ebenso möglich, den erfindungsgemäßen Superabsorber durch Wasserzusatz auf einen gewünschten Wassergehalt einzustellen.

Wahlweise werden die erfindungsgemäßen Superabsorber mit weiteren Zusätzen versehen, die gegen Verfärbung stabilisieren. Beispiele sind insbesondere bekannte Stabilisatoren gegen Verfärbung, insbesondere reduzierende Substanzen. Unter diesen Sind fest oder gelöste Salze der Phosphinsäure (H₃PO₂) sowie diese selbst bevorzugt. Beispielsweise eignen sich alle Phosphinate der Alkalimetalle, inklusive des Ammoniums, und der Erdalkalimetalle. Besonders bevorzugt sind wässrige Lösungen der Phosphinsäure welche Phosphinationen sowie mindestens ein Kation ausgewählt aus Natrium, Kalium, Ammonium, Calcium, Strontium, Aluminium, Magnesium enthalten. Ebenso bevorzugt sind Salze der Phosphonsäure (H₃PO₃) sowie diese selbst. Beispielsweise eignen sich alle primären und sekundären Phosphonate der Alkalimetalle, inklusive des Ammoniums, und der Erdalkalimetalle. Besonders bevorzugt sind wässrige Lösungen der Phosphonsäure, welche primäre und/oder sekundäre Phosphonationen sowie mindestens ein Kation ausgewählt aus Natrium, Kalium, Calcium, Strontium enthalten.

Alle Beschichtungen, Feststoffe, Zusätze und Hilfsstoffe können jeweils in separaten Verfahrensschritten zugegeben werden, meist ist jedoch die bequemste Methode, sie - falls sie nicht während der Versetzung des Grundpolymers mit Oberflächennachvernetzungsmittel zugegeben werden - dem Superabsorber im Kühler zuzugeben, etwa durch Aufsprühen einer Lösung oder Zugabe in feinteiliger fester oder in flüssiger Form.

Der L-Wert des Superabsorbers (CIE-Farbzahl) beträgt im nicht gelagerten Zustand typischerweise mindestens 75, vorzugsweise mindestens 80, besonders bevorzugt mindestens 85 und höchstens 100.

Der α-Wert des Superabsorbers (CIE-Farbzahl) beträgt im nicht gelagerten Zustand typischerweise von -2,5 bis +2,5, vorzugsweise von -2,0 bis +2,0, besonders bevorzugt von -1,5 bis +1,5.

Der b-Wert des Superabsorbers (CIE-Farbzahl) beträgt im nicht gelagerten Zustand typischerweise von 0 bis 10.

Ein weiterer Gegenstand der vorliegenden Erfindung ist der mit dem erfindungsgemäßen Verfahren erhältliche Superabsorber, der auch und gerade bei relativ hohem Gehalt der Monomermischung an Eisenionen einen verglichen mit anderen Superabsorbern niedrigen Restmonomerengehalt aufweist und dabei auch vergleichsweise farblos ist.

Der erfindungsgemäße Superabsorber hat im Allgemeinen eine Zentrifugenretentionskapazität (CRC) von mindestens 5 g/g, vorzugsweise von mindestens 10 g/g und in besonders bevorzugter Form von mindestens 20 g/g. Weitere geeignete Mindestwerte der CRC sind beispielsweise 25 g/g, 30 g/g oder 35 g/g. Üblicherweise liegt sie nicht über 40 g/g. Ein typischer Bereich der CRC für oberflächennachvernetzte Superabsorber ist von 28 bis 33 g/g.

Der erfindungsgemäße Superabsorber hat, wenn er oberflächennachvernetzt ist, typischerweise eine Absorption unter Druck (AUL0.7psi, Messmethode s. unten) von mindestens 18 g/g, vorzugsweise mindestens 20 g/g, bevorzugt mindestens 22 g/g, besonders bevorzugt mindestens 23 g/g, ganz besonders bevorzugt mindestens 24 g/g und üblicherweise nicht über 30 g/g.

Der erfindungsgemäße Superabsorber hat weiterhin eine Flüssigkeitsweiterleitung (SFC, Messmethode s. unten) von mindestens 10x10⁻⁷cm³s/g, vorzugsweise mindestens 30x10⁻⁷cm³s/g, bevorzugt mindestens 50x10⁻⁷cm³s/g, besonders bevorzugt mindestens 80x10⁻⁷cm³s/g, ganz besonders bevorzugt mindestens 100x10⁻⁷cm³s/g und üblicherweise nicht über 1000x10⁻⁷cm³s/g.

Ein weiterer Gegenstand der vorliegenden Erfindung sind Hygieneartikel, enthaltend erfindungsgemäße Superabsorber, vorzugsweise ultradünne Windeln, enthaltend eine absorbierende Schicht bestehend aus 50 bis 100 Gew.-%, vorzugsweise 60 bis 100 Gew.-%, bevorzugt 70 bis 100 Gew.-%, besonders bevorzugt 80 bis 100 Gew.-%, ganz besonders bevorzugt 90 bis 100 Gew.-%, erfindungsgemäßer Superabsorber, wobei die Umhüllung der absorbierenden Schicht selbstverständlich nicht berücksichtigt ist.

Ganz besonders vorteilhaft sind die erfindungsgemäßen Superabsorber auch zur Herstellung von Laminaten und Kompositstrukturen, wie sie beispielsweise in der US 2003/0181115 sowie der US 2004/0019342 beschrieben sind, geeignet. Zusätzlich zu den in beiden Schriften zur Herstellung solcher neuer absorbierenden Strukturen beschriebenen Schmelzklebern und insbesondere den in der US 2003/0181115 beschriebenen Fasern aus Schmelzklebern, an die die Superabsorberpartikel gebunden sind, eignen sich die erfindungsgemäßen Superabsorber auch zur Herstellung von vollkommen analogen Strukturen unter Verwendung von UV-vernetzbaren Schmelzklebern, welche beispielsweise als AC-Resin^{®} (BASF SE, Carl-Bosch-Straße 38, 67056 Ludwigshafen, Deutschland) vertrieben werden. Diese UV-vernetzbaren Schmelzkleber haben den Vorteil bereits bei 120 bis 140 °C verarbeitbar zu sein, daher sind sie mit vielen thermoplastischen Substraten besser kompatibel. Ein weiterer wesentlicher Vorteil besteht darin, dass UV-vernetzbare Schmelzkleber toxikologisch sehr unbedenklich sind und auch keine Ausdünstungen in den Hygieneartikeln verursachen. Ein ganz wesentlicher Vorteil, im Zusammenhang mit den erfindungsgemäßen Superabsorbern, ist die Eigenschaft der UV-vernetzbaren Schmelzkleber während der Verarbeitung und Vernetzung nicht zur Vergilbung zu neigen. Dies ist insbesondere von Vorteil, wenn ultradünne oder teilweise transparente Hygieneartikel hergestellt werden sollen. Die Kombination der erfindungsgemäßen Superabsorber mit UV-vernetzbaren Schmelzklebern ist daher besonders vorteilhaft. Geeignete UV-vernetzbare Schmelzkleber sind beispielsweise beschrieben in EP 0 377 199 A2, EP 0 445 641 A1, US 5,026,806, EP 0 655 465 A1 und EP 0 377 191 A2.

Der erfindungsgemäße Superabsorber kann außerdem in anderen Gebieten der Technik eingesetzt werden, bei denen Flüssigkeiten, insbesondere Wasser oder wässrige Lösungen absorbiert werden. Diese Gebiete sind beispielsweise Lagerung, Verpackung, Transport (als Bestandteile von Verpackungsmaterial für wasser- oder feuchtigkeitsempfindliche Artikel, etwa zum Blumentransport, auch als Schutz gegen mechanische Einwirkungen); Tierhygiene (in Katzenstreu); Lebensmittelverpackung (Transport von Fisch, Frischfleisch; Absorption von Wasser, Blut in Frischfisch- oder - fleischverpackungen); Medizin (Wundpflaster, wasserabsorbierendes Material für Brandverbände oder für andere nässende Wunden), Kosmetik (Trägermaterial für Pharmachemikalien und Medikamente, Rheumapflaster, Ultraschallgel, Kühlgel, Kosmetikverdicker, Sonnenschutz); Verdicker für Öl/Wasser bzw. Wasser/Öl-Emulsionen; Textilien (Feuchtigkeitsregulation in Textilien, Schuheinlagen, zur Verdampfungskühlung, etwa in Schutzkleidung, Handschuhen, Stirnbändern); chemisch-technische Anwendungen (als Katalysator für org. Reaktionen, zur Immobilisierung großer funktioneller Moleküle wie Enzymen, als Adhäsionsmittel bei Agglomerationen, Wärmespeicher, Filtrationshilfsmittel, hydrophile Komponente in Polymerlaminaten, Dispergiermittel, Verflüssiger); als Hilfsmittel beim Pulverspritzguss, im Bau- und Konstruktionswesen (Installation, in lehmbasierenden Putzen, als vibrationshemmendes Medium, Hilfsmittel bei Tunnelgrabungen in wasserreichem Untergrund, Kabelummantelung); Wasserbehandlung, Abfallbehandlung, Wasserabtrennung (Enteisungsmittel, wiederverwendbare Sandsäcke); Reinigung; Agrarindustrie (Bewässerung, Rückhaltung von Schmelzwasser und Tauniederschläge, Kompostierungszusatz, Schutz der Wälder vor Pilz-/Insektenbefall, verzögerte Freisetzung von Wirkstoffen an Pflanzen); zur Brandbekämpfung oder zum Brandschutz; Coextrusionsmittel in thermoplastischen Polymeren (z. B. zur Hydrophilierung von Mehrschichtfolien); Herstellung von Folien und thermoplastischen Formkörpern, die Wasser absorbieren können (z.B. Regen- und Tauwasser speichernde Folien für die Landwirtschaft; Superabsorber enthaltende Folien zum Frischhalten von Obst und Gemüse, die in feuchte Folien verpackt werden; Superabsorber-Polystyrol Coextrudate, beispielsweise für Lebensmittelverpackungen wie Fleisch, Fisch, Geflügel, Obst und Gemüse); oder als Trägersubstanz in Wirkstoffformulierungen (Pharma, Pflanzenschutz).

Die erfindungsgemäßen Artikel zur Absorption von Flüssigkeit unterscheiden sich von bekannten dadurch, dass sie den erfindungsgemäßen Superabsorber enthalten.

Es wurde außerdem ein Verfahren zur Herstellung von Artikeln zur Absorption von Flüssigkeit, insbesondere Hygieneartikeln gefunden, das dadurch gekennzeichnet ist, dass man bei der Herstellung des betreffenden Artikels mindestens einen erfindungsgemäßen Superabsorber einsetzt. Im übrigen sind Verfahren zur Herstellung solcher Artikel unter Einsatz von Superabsorber bekannt.

### Testmethoden

Der Superabsorber wird mit den nachfolgend beschriebenen Testmethoden geprüft.

Die nachfolgend beschriebenen, mit "WSP" bezeichneten Standard-Testmethoden werden beschrieben in: "Standard Test Methods for the Nonwovens Industry", Ausgabe 2005, gemeinsam herausgegeben von den "Worldwide Strategic Partners" EDANA (European Disposables and Nonwovens Association, Avenue Eugène Plasky, 157, 1030 Brussels, Belgium, www.edana.org) und INDA (Association of the Nonwoven Fabrics Industry, 1100 Crescent Green, Suite 115, Cary, North Carolina 27518, U.S.A., www.inda.org). Diese Veröffentlichung ist sowohl von EDANA als auch von INDA erhältlich.

Alle nachfolgend beschriebenen Messungen sollten, wenn nicht anders angegeben, bei einer Umgebungstemperatur von 23 ± 2 °C und einer relativen Luftfeuchte von 50 ± 10 % durchgeführt werden. Die Superabsorberpartikel werden vor der Messung gut durchmischt, wenn nicht anders angegeben.

### Zentrifugenretentionskapazität (CRC, Centrifuge Retention Capacity)

Die Zentrifugenretentionskapazität des Superabsorbers wird gemäß der Standard-Testmethode Nr. WSP 241.5-02 "Centrifuge retention capacity" bestimmt.

### Absorption unter Druck (AUL0.3psi, "Absorbency Under Load of 0.3 psi")

Die Absorption unter einem Druck von 2068 Pa (0.3 psi) des Superabsorbers wird gemäß der Standard-Testmethode Nr. WSP 242.2-05 "Absorption under pressure" bestimmt.

### Absorption unter Druck (AUL0.7psi, "Absorbency Under Load of 0.7 psi)

Die Absorption unter einem Druck von 4826 Pa (0.7 psi) des Superabsorbers wird analog der Standard-Testmethode Nr. WSP 242.2-05 "Absorption under pressure" bestimmt, wobei jedoch ein Gewicht mit 49 g/cm² (führt zu einem Druck von 0.7 psi) statt eines Gewichts mit 21 g/cm² (führt zu einem Druck von 0.3 psi) verwendet wird.

### Feuchtegehalt des Superabsorbers (Restfeuchte, Wassergehalt)

Der Wassergehalt der Superabsorberpartikel wird gemäß der Standard-Testmethode Nr. WSP 230.2-05 "Moisture content" bestimmt.

### Mittlere Partikelgröße

Die mittlere Partikelgröße der Produktfraktion wird gemäß der Standard-Testmethode Nr. WSP 220.2-05 "Partikel size distribution" ermittelt.

### Restmonomerengehalt

Der Gehalt der Superabsorberpartikel an Restmonomeren wird gemäß der Standard-Testmethode Nr. WSP 210.2-05 "Residual Monomers" bestimmt.

### CIE-Farbzahl (L a b)

Die Farbmessung wird entsprechend dem CIELAB-Verfahren (Hunterlab, Band 8, Jahrgang 1996, Heft 7, Seiten 1 bis 4) mit einem Colorimeter, Modell "LabScan XE S/N LX17309" (HunterLab, Reston, U.S.A.)durchgeführt. Dabei werden die Farben über die Koordinaten L, a und b eines dreidimensionalen Systems beschrieben. Dabei gibt L die Helligkeit an, wobei L = 0 schwarz und L = 100 weiß bedeutet. Die Werte für a und b geben die Position der Farbe auf den Farbachsen rot/grün bzw. gelb/blau an, wobei +a für rot, -a für grün, +b für gelb und -b für blau steht.

Die Farbmessung entspricht dem Dreibereichsverfahren nach DIN 5033-6.

### Beispiele

### Beispiel 1: Herstellung eines Grundpolymers (Vergleich)

Ein Kneter mit zwei Sigma-Wellen (Modell LUK 8.0 K2, hergestellt von Coperion Werner & Pfleiderer GmbH & Co. KG, Stuttgart, Deutschland) wurde zur Inertisierung mit Stickstoff durchspült und danach mit einer durch Durchperlen von Stickstoff von Sauerstoff befreiten Mischung aus 5166 g einer 37,7 Gew.-%igen wässrigen Natriumacrylatlösung, 574 g Acrylsäure und 720 g entsalztem Wasser gefüllt. Anschließend wurden 6,2 g 3-fach ethoxyliertes Glycerintriacrylat als Innenvernetzer sowie im Anschluss daran als Initiator 16,5 g einer 0,75 Gew.-%igen wässrigen Ascorbinsäurelösung, 38,6 g einer 15 Gew.-%igen wässrigen Natriumpersulfatlösung und 3,4 g einer 3 Gew.-%igen wässrigen Wasserstoffperoxidlösung zugefügt. Der Kneter wurde mit Geschwindigkeiten von 98 Umdrehungen pro Minute an der einen Welle und mit 49 Umdrehungen pro Minute an der anderen Welle betrieben. Unmittelbar nach der Zugabe der Wasserstoffperoxidlösung wurde die Lösung mittels Durchleiten von Heizflüssigkeit (80 °C) durch den Heizmantel des Kneters erwärmt. Sobald die Temperatur im Kneter nicht weiter anstieg wurde die Beheizung beendet und das Polymergel für weitere 14 Minuten reagieren gelassen. Anschließend wurde das Gel auf etwa 65 °C abkühlen gelassen und aus dem Kneter entnommen. Das Gel wurde in Portionen von je 400 g mit einem Umlufttrockner bei 160 °C für 22 Minuten getrocknet, wobei das Gel in einem Siebeinsatz trocknete, der von unten mit Luft dieser Temperatur durchströmt wurde, welche durch ein Heißluftgebläse erzeugt wurde. Die heiße Luft strömt während dieser Trocknung von unten nach oben durch das Gel. Abschließend wurde das getrocknete Gel dreimal auf einem Walzenstuhl gemahlen (Modell LRC 125/70, hergestellt von Bauermeister Zerkleinerungstechnik GmbH, Norderstedt, Deutschland),wobei nacheinander Spaltweiten von 1000 µm, 600 µm und 400 µm eingestellt wurden. Der Superabsorber wurde abgesiebt, wobei der Siebschnitt von 300 bis 600 µm als Grundpolymer gewonnen wurde.

### Beispiel 2 (Vergleich)

Beispiel 1 wurde wiederholt, wobei der Monomermischung 10,3 g Harnstoff (= 0,5 Gew.% bezogen auf Acrylsäuremonomeres: das Natriumacrylat wird für die Berechnung in Acrylsäure umgerechnet und zur freien Acrylsäure addiert. Bei allen weiteren Mengenangaben unten wurde ebenso gerechnet.) zugesetzt wurden.

### Beispiel 3 (Vergleich)

Beispiel 1 wurde wiederholt, wobei der Monomermischung 20,7 g Harnstoff zugesetzt wurden (= 1,0 Gew.% bezogen auf Acrylsäuremonomeres).

### Beispiel 4

Beispiel 1 wurde wiederholt, wobei der Monomermischung 10,3 g Harnstoffphosphat zugesetzt wurden (= 0,5 Gew.% bezogen auf Acrylsäuremonomeres).

### Beispiel 5

Beispiel 1 wurde wiederholt, wobei der Monomermischung 20,7 g Harnstoffphosphat zugesetzt wurden (= 1,0 Gew.% bezogen auf Acrylsäuremonomeres).

### Beispiel 6

Beispiel 5 wurde wiederholt, wobei der Monomermischung zusätzlich 15 Gew.-ppm Eisen(II)sulfat (FeSO₄ · 7 H₂O) bezogen auf Acrylsäuremonomeres zugesetzt wurden (= 3 Gew.-ppm Eisenionen bezogen auf Acrylsäuremonomeres).

### Beispiel 7

Beispiel 5 wurde wiederholt, wobei der Monomermischung zusätzlich 50 Gew.-ppm Eisen(II)sulfat (FeSO₄ · 7 H₂O) bezogen auf Acrylsäuremonomeres zugesetzt wurden (= 10 Gew.-ppm Eisenionen bezogen auf Acrylsäuremonomeres).

### Beispiel 8 Herstellung eines Grundpolymers (Vergleich)

Ein Kneter mit zwei Sigma-Wellen (Modell LUK 8.0 K2, hergestellt von Coperion Werner & Pfleiderer GmbH & Co. KG, Stuttgart, Deutschland) wurde zur Inertisierung mit Stickstoff durchspült und danach mit einer durch Durchperlen von Stickstoff von Sauerstoff befreiten Mischung aus 4701 g einer 37,7 Gew.-%igen wässrigen Natriumacrylatlösung, 522 g Acrylsäure und 1237 g entsalztem Wasser gefüllt. Anschließend wurden 4,7 g 3-fach ethoxyliertes Glycerintriacrylat als Innenvernetzer sowie im Anschluss daran als Initiator 15,0 g einer 0,75 Gew.-%igen wässrigen Ascorbinsäurelösung, 35,1 g einer 15 Gew.-%igen wässrigen Natriumpersulfatlösung und 3,1 g einer 3 Gew.-%igen wässrigen Wasserstoffperoxidlösung zugefügt. Der Kneter wurde mit Geschwindigkeiten von 98 Umdrehungen pro Minute an der einen Welle und mit 49 Umdrehungen pro Minute an der anderen Welle betrieben. Unmittelbar nach der Zugabe der Wasserstoffperoxidlösung wurde die Lösung mittels Durchleiten von Heizflüssigkeit (80 °C) durch den Heizmantel des Kneters erwärmt. Sobald die Temperatur im Kneter nicht weiter anstieg wurde die Beheizung beendet und das Polymergel für weitere 14 Minuten reagieren gelassen. Anschließend wurde das Gel auf etwa 65 °C abkühlen gelassen und aus dem Kneter entnommen. Das Gel wurde in Portionen von je 400 g mit einem Umlufttrockner bei 160 °C für 22 Minuten getrocknet, wobei das Gel in einem Siebeinsatz trocknete, der von unten mit Luft dieser Temperatur durchströmt wurde, welche durch ein Heißluftgebläse erzeugt wird. Die heiße Luft strömt während dieser Trocknung von unten nach oben durch das Gel. Abschließend wurde das getrocknete Gel dreimal auf einem Walzenstuhl gemahlen (Modell LRC 125/70, hergestellt von Bauermeister Zerkleinerungstechnik GmbH, Norderstedt, Deutschland), wobei nacheinander Spaltweiten von 1000 µm, 600 µm und 400 µm eingestellt wurden. Der Superabsorber wurde abgesiebt, wobei der Siebschnitt von 300 bis 600 µm als Grundpolymer gewonnen wurde.

### Beispiel 9 (Vergleich)

Beispiel 8 wurde wiederholt, wobei der Monomermischung zusätzlich 15 Gew.-ppm Eisen(II)sulfat (FeSO₄ · 7 H₂O) bezogen auf Acrylsäuremonomeres zugesetzt wurden (= 3 Gew.-ppm Eisenionen bezogen auf Acrylsäuremonomeres).

### Beispiel 10 (Vergleich)

Beispiel 8 wurde wiederholt, wobei der Monomermischung zusätzlich 15 Gew.-ppm Eisen(II)sulfat (FeSO₄ · 7 H₂O) bezogen auf Acrylsäuremonomeres zugesetzt wurden (= 3 Gew.-ppm Eisenionen bezogen auf Acrylsäuremonomeres), und es wurden zusätzlich noch 29 Gew.-ppm KH₂PO₄ bezogen auf Acrylsäuremonomeres zugesetzt.

Die Eigenschaften der Superabsorber aus den Beispielen 1 bis 10 sind in Tabelle 1 zusammengefasst.

Der Vergleich der Beispiele in Tabelle 1 zeigt, dass der Zusatz von Harnstoff den Restmonomerengehalt erst ab einer Menge von 1 Gew.-% bezogen auf Acrylsäuremonomeres (= bzAsm, s. Erläuterung zur Tabelle) auf ein erwünschtes Maß senkt (Beispiele 1, 2 und 3), während Harnstoffphosphat dies bereits ab einer Menge von 0,5 Gew.-% bzAsm erreicht (Beispiele 1, 4 und 5). Allerdings zeigt der Superabsorber mit 1,0 Gew.% bzAsm Harnstoffzusatz eine tendenziell stärkere unerwünschte Gelbfärbung (erhöhte b-Farbzahl). Weiterhin wird deutlich, dass Eisenverunreinigungen den Restmonomerengehalt erhöhen (Beispiele 8 und 9) und zu tendenziell stärkerer Gelbfärbung führen (Beispiele 8 und 9 sowie 1, 6 und 7). Der Zusatz von Phosphat beeinflusst weder den Restmonomerengehalt noch die Gelbfärbung (Beispiele 9 und 10). Die erfindungsgemäßen Beispiele zeigen, dass Harnstoffphosphat im Gegensatz zu den Komponenten Harnstoff und Phosphat schon in vergleichsweise niedrigen Mengen und auch bei Vorhandensein von Eisenverunreinigungen den Restmonomerengehalt sehr deutlich absenkt, wenn auch in Anwesenheit von Eisen eine tendenziell stärkere Gelbfärbung auftritt.

### Beispiel 11 (Vergleich): Herstellung eines oberflächennachvernetzten Superabsorbers

In einer Küchenmaschine (Hersteller: Robert Bosch GmbH, Robert-Bosch-Platz 1; 70839 Gerlingen-Schillerhöhe, Deutschland, Modell "Profimixx 47 / MUM4700") mit Schneebesen wurden 250 g des Polymers von Beispiel 1 eingefüllt und auf Stufe 4 durchmischt. 10 g einer Lösung aus 6,82 g vollentsalztem Wasser, 2,92 g Isopropanol, 0,25 g einer Mischung aus gleichen Gewichts-Teilen 1,3-Propandiol und N-(2-Hydroxiethyl)-2-oxazolidinon und 0,010 g Sorbitanmonococoat ("Span^{®} 20") wurden über eine Zweistoffdüse (Büchi Labortechnik GmbH, Am Porscheplatz 5, 45127 Essen, Deutschland, www.buechigmbh.de; Artikel Nr. 004364, 0,7 mm); betrieben mit Stickstoff (0,25 bar Überdruck) als Trägergas binnen 20 Sekunden auf das durchmischte Grundpolymer aufgesprüht, danach wurde für weitere 2 Minuten durchmischt. Der Motor wurde angehalten und an der Rührschüsselwand und dem Werkzeug anhaftendes Polymer wieder gelöst. Anschließend wurde der Rührschüsselinhalt nochmals für weitere 100 Sekunden durchmischt.

Das Polymer wurde auf ein Blech gegeben, möglichst homogen ausgebreitet und für 45 Minuten in einem auf 200 °C vorgeheizten Umluft-Trockenschrank getrocknet. Nach Abkühlung auf Raumtermperatur wurden aus dem oberflächennachvernetzten Superabsorber Agglomerate und Grobpartikel mittels eines Siebes von 850 µm Maschenweite entfernt.

### Beispiel 12

Beispiel 11 wurde mit dem Polymer von Beispiel 2 wiederholt.

### Beispiel 13

Beispiel 11 wurde mit dem Polymer von Beispiel 3 wiederholt.

### Beispiel 14

Beispiel 11 wurde mit dem Polymer von Beispiel 4 wiederholt.

### Beispiel 15

Beispiel 11 wurde mit dem Polymer von Beispiel 5 wiederholt.

### Beispiel 16

Beispiel 11 wurde mit dem Polymer von Beispiel 6 wiederholt.

### Beispiel 17

Beispiel 11 wurde mit dem Polymer von Beispiel 7 wiederholt.

### Beispiel 18

In einem Labormischer (Hersteller Waring Products, Inc., Torrington, Connecticut, U.S.A., Modell 34 BL 99 (8012)) mit zwei gegenüberstehenden abgerundeten Mischblättern und Wellenbrecher am Deckel (vergleichbare Ergebnisse werden auch in vielen anderen Mischern mit guter Durchmischung während der Aufgabe der Nachvernetzungslösung erzielt, wobei darauf zu achten ist, dass die Rührorgane den Superabsorber nicht zerkleinern - entsprechend ist die Rührgeschwindigkeit einzustellen) wurden 20 g des Polymers von Beispiel 8 vorgelegt. Aus einer Einmalspritze wurden auf das durchmischte Polymer bei mittlerer Rührstufe des Mischers eine Mischung aus 0,3 g Isopropanol, 0,7 g Wasser und 20 mg Ethylenglykoldiglycidylether (Denacol^{®} EX-810 von Nagase ChemteX Corporation, Osaka, Japan) getropft.

Das Polymer wurde anschließend auf einem Uhrglas bei 150 °C für eine Stunde im Umlufttrockenschrank getrocknet und schließlich zur Abtrennung von Klumpen durch ein 850 µm-Sieb gesiebt.

### Beispiel 19

Beispiel 18 wurde mit dem Polymer von Beispiel 9 wiederholt.

### Beispiel 20

Beispiel 18 wurde mit dem Polymer von Beispiel 10 wiederholt.

Die Eigenschaften der Superabsorber aus den Beispielen 11 bis 20 sind in Tabelle 2 zusammengefasst.

Der Vergleich der Beispiele in Tabelle 2 zeigt, dass weder ein Zusatz von Harnstoff, Phosphat oder Harnstoffphosphat einen signifikanten Einfluss auf die Oberflächennachvernetzung zeigt. Die Superabsorber von Beispielen 1 bis 10 werden durch die Oberflächennachvernetzung lediglich so verändert, wie für die jeweils durchgeführte Oberflächennachvernetzung zu erwarten ist.

**Tabelle 1: Nicht oberflächennachvernetzte Superabsorber**

| | | | | | | | Farbzahlen | | |
|---|---|---|---|---|---|---|---|---|---|
| Beispiel | Harnstoffphosphat [Gew.-% bzAsm] | Fe* [Gew.-% bzAsm] | Sonstiges [Gew.-% bzAsm ] | CRC [g/g] | AUL 0.3 psi [g/g] | Restmonomere [Gew.-ppm] | L | a | b |
| 1 (Vergleich) | - | - | - | 35,7 | 13,1 | 1040 | 92,6 | -0,5 | 4,8 |
| 2 (Vergleich) | - | - | Harnstoff, 0,5 | 36,0 | 14,5 | 1120 | 92,3 | -0,8 | 6,7 |
| 3 (Vergleich) | - | - | Harnstoff, 1,0 | 35,5 | 19,4 | 380 | 91,9 | -0,7 | 7,4 |
| 4 | 0,5 | - | - | 33,6 | 15,9 | 390 | 93,0 | -0,8 | 6,1 |
| 5 | 1,0 | - | - | 34,2 | 13,7 | 370 | 92,4 | -0,8 | 6,6 |
| 6 | 1,0 | 0,0003 | - | 35,4 | 12,0 | 220 | 91,0 | -0,3 | 8,5 |
| 7 | 1,0 | 0,0010 | - | 36,0 | 9,7 | 370 | 89,9 | 0,3 | 8,2 |
| 8 (Vergleich) | - | - | - | 39,1 | 8,4 | 1660 | 92,7 | -0,5 | 4,9 |
| 9 (Vergleich) | - | 0,0003 | - | 40,5 | 7,6 | 2860 | 90,8 | -0,2 | 5,6 |
| 10 (Vergleich) | - | 0,0003 | KH₂PO₄, 0,0029 | | | 2840 | 90,2 | -0,1 | 5,9 |

| | | | | | | | | | |
|---|---|---|---|---|---|---|---|---|---|
| "bzAsm" ("bezogen auf Acrylsäuremonomere"): berechnet bezogen auf die Masse der eingesetzten Acrylsäure, wobei das eingesetzte Natriumacrylat als Acrylsäure berechnet wird, d.h. für die Masse jedes Mols Natriumacrylat wird die Masse eines Mols Acrylsäure berechnet. "n.b.": nicht bestimmt *) das Eisen wird als Eisen(II)sulfatheptahydrat eingesetzt, die Tabelle gibt die resultierende Einsatzmenge an Eisen bezogen auf berechnetes Acrylsäuremonomeres an. | | | | | | | | | |

**Tabelle 2: Oberflächennachvernetzte Superabsorber**

| | | | | | | Farbzahlen | | |
|---|---|---|---|---|---|---|---|---|
| Beispiel | Harnstoffphosphat [Gew.-% bzAsm] | Fe* [Gew.-% bzAsm] | sonst. [Gew.-% bzAsm] | CRC [g/g] | AUL 0.7 psi [g/g] | L | a | b |
| 11 (Vergleich) | - | - | - | 34 | 25 | 93,1 | -1,0 | 5,8 |
| 12 (Vergleich) | - | - | Harnstoff, 0,5 | 32 | 25 | 92,3 | -1,1 | 7,2 |
| 13 (Vergleich) | - | - | Harnstoff, 1,0 | 30 | 25 | 91,4 | -0,6 | 8,2 |
| 14 | 0,5 | - | - | 31 | 25 | 92,4 | -1,0 | 6,6 |
| 15 | 1,0 | - | - | 31 | 25 | 92,5 | -0,9 | 7,2 |
| 16 | 1,0 | 0,0003 | - | 30 | 23 | 90,8 | -0,4 | 9,2 |
| 17 | 1,0 | 0,0010 | - | 30 | 24 | 90,0 | 0,2 | 9,0 |
| 18 (Vergleich) | - | - | - | 35,3 | 26,5 | n.b. | n.b. | n.b. |
| 19 (Vergleich) | - | 0,0003 | - | 36,4 | 23,6 | n.b. | n.b. | n.b. |
| 20 (Vergleich) | - | 0,0003 | KH₂PO₄, 0,0029 | 36,2 | 24,1 | n.b. | n.b. | n.b. |

| | | | | | | | | |
|---|---|---|---|---|---|---|---|---|
| "bzAsm": siehe Anmerkung zu Tabelle 1, "n.b.": nicht bestimmt *) das Eisen wird als Eisen(II)sulfatheptahydrat eingesetzt, die Tabelle gibt die resultierende Einsatzmenge an Eisen bezogen auf berechnetes Acrylsäuremonomeres an. | | | | | | | | |

## Patentansprüche

1. Polymerisationsverfahren zur Herstellung von Superabsorbern, wobei man der Monomermischung vor oder während der Polymerisation, oder dem Polymeren nach der Polymerisation, aber vor einer der Polymerisation folgenden Wärmebehandlung ein Salz des Harnstoffs mit einer anorganischen Säure zugibt.

2. Verfahren nach Anspruch 1, **dadurch gekennzeichnet, dass** man ein Salz des Harnstoffs mit Schwefelsäure, Phosphorsäure oder einer Halogenwasserstoffsäure zugibt.

3. Verfahren nach Anspruch 2, **dadurch gekennzeichnet, dass** man Harnstoffphosphat zugibt.

4. Verfahren nach Anspruch 1, **dadurch gekennzeichnet, dass** man eine wässrige Lösung einer Monomermischung polymerisiert, die enthält:
a) mindestens ein ethylenisch ungesättigtes, säuregruppentragendes Monomer, das wahlweise zumindest teilweise als Salz vorliegt,
b) mindestens einen Vernetzer,
c) mindestens einen Initiator,
d) wahlweise ein oder mehrere mit den unter a) genannten Monomeren copolymerisierbare ethylenisch ungesättigte Monomere,
e) wahlweise ein oder mehrere wasserlösliche Polymere und
f) ein Salz des Harnstoffs mit einer anorganischen Säure.

5. Verfahren nach Anspruch 4, **dadurch gekennzeichnet, dass** die anorganische Säure Schwefelsäure, Phosphorsäure oder eine Halogenwasserstoffsäure ist.

6. Verfahren nach Anspruch 5, **dadurch gekennzeichnet, dass** die anorganische Säure Phosphorsäure ist.

7. Superabsorber, erhältlich nach einem der in den Ansprüchen 1 bis 6 definierten Verfahren.

8. Superabsorber nach Anspruch 7, **dadurch gekennzeichnet, dass** er oberflächennachvernetzt ist.

9. Artikel zur Absorption von Flüssigkeiten, enthaltend einen der in Ansprüchen 7 oder 8 definierten Superabsorber.

10. Verfahren zur Herstellung von Artikeln zur Absorption von Flüssigkeit, **dadurch gekennzeichnet, dass** man bei der Herstellung einen in Ansprüchen 7 oder 8 definierten Superabsorber einsetzt.

## Claims

1. A polymerisation process for preparing superabsorbents, by adding a salt of urea with an inorganic acid to the monomer mixture before or during the polymerization, or to the polymer after the polymerisation but before a heat treatment which follows the polymerization.

2. The process according to claim 1, wherein a salt of urea with sulfuric acid, phosphoric acid or a hydrohalic acid is added.

3. The process according to claim 2, wherein urea phosphate is added.

4. The process according to claim 1, wherein an aqueous solution of a monomer mixture is polymerized, said monomer mixture comprising:
a) at least one ethylenically unsaturated monomer which bears acid groups and is optionally present at least partly in salt form,
b) at least one crosslinker,
c) at least one initiator,
d) optionally one or more ethylenically unsaturated monomers copolymerizable with the monomers specified under a),
e) optionally one or more water-soluble polymers and
f) a salt of urea with an inorganic acid.

5. The process according to claim 4, wherein the inorganic acid is sulfuric acid, phosphoric acid or a hydrohalic acid.

6. The process according to claim 5, wherein the inorganic acid is phosphoric acid.

7. A superabsorbent obtainable by any one of the processes defined in claims 1 to 6.

8. The superabsorbent according to claim 7, which is surface postcrosslinked.

9. An article for adsorbing fluids, comprising any one of the superabsorbents defined in claims 7 and 8.

10. A process for producing articles a for absorbing fluid, which comprises using a superabsorbent defined in claims 7 and 8 in said production.

## Revendications

1. Procédé de polymérisation pour la production de superabsorbants, dans lequel on ajoute au mélange de monomères avant ou pendant la polymérisation, ou au polymère après la polymérisation, mais avant un traitement thermique faisant suite à la polymérisation, un sel de l'urée avec un acide inorganique.

2. Procédé selon la revendication 1, **caractérisé en ce qu'**on ajoute un seul de l'urée avec l'acide sulfurique, l'acide phosphorique ou un hydracide halogéné.

3. Procédé selon la revendication 2, **caractérisé en ce qu'**on ajoute du phosphate d'urée.

4. Procédé selon la revendication 1, **caractérisé en ce qu'**on polymérise une solution aqueuse d'un mélange de monomères qui contient :
a) au moins un monomère à insaturation éthylénique, pourtant des groupes acides, qui en option peut se trouver au moins en partie sous forme de sel,
b) au moins un agent de réticulation,
c) au moins un amorceur,
d) en option un ou plusieurs monomères à insaturation éthylénique, copolymérisables avec les monomères nommés en a),
e) en option un ou plusieurs polymères hydrosolubles et
f) un sel de l'urée avec un acide inorganique.

5. Procédé selon la revendication 4, **caractérisé en ce que** l'acide inorganique est l'acide sulfurique, l'acide phosphorique ou un hydracide halogéné.

6. Procédé selon la revendication 5, **caractérisé en ce que** l'acide inorganique est l'acide phosphorique.

7. Superabsorbant, pouvant être obtenu selon l'un des procédés définis dans les revendications 1 à 6.

8. Superabsorbant selon la revendication 7, **caractérisé en ce qu'**il est post-réticulé en surface.

9. Article destiné à l'absorption de liquides, contenant l'un des superabsorbants définis dans la revendication 7 ou 8.

10. Procédé pour la fabrication d'articles destinés à l'absorption de liquide, **caractérisé en ce que** dans la fabrication on utilise un superabsorbant défini dans la revendication 7 ou 8.
